# EUROPEAN PATENT SPECIFICATION

(11) **EP 1 465 584 B1**
(45) Date of publication and mention of the grant of the patent: **23.01.2008**
(21) Application number: 02805972.3
(22) Date of filing: 20.12.2002
(51) Int. Cl.: A61Q 5/02, A61Q 19/10, C11D 1/94, C11D 17/00

(54) **COMBINED STABLE CATIONIC AND ANIONIC SURFACTANT COMPOSITIONS**
KOMBINIERTE STABILE KATIONISCHE UND ANIONISCHE TENSIDZUSAMMENSETZUNGEN
COMPOSITIONS STABLES ASSOCIANT DES TENSIOACTIFS CATIONIQUES ET DES TENSIOACTIFS ANIONIQUES

(30) Priority: 21.12.2001 US 341810 P; 01.04.2002 US 369219 P
(43) Date of publication of application: 13.10.2004
(73) Proprietor: RHODIA INC., Cranbury, NJ 08512 (US)
(72) Inventor: FRANTZ, Seren, Bensalem, PA 19020 (US); WARBURTON, Stewart, West Windsor, NJ 08550 (US)
(74) Representative: Kyle, Diana
(86) International application number: PCT/US2002/041125
(87) International publication number: WO 2003/055455

(56) References cited:
- EP-A- 0 825 200
- WO-A-01/24807
- GB-A- 2 292 155
- US-A- 4 997 641
- US-A- 5 358 667
- US-A- 6 150 312
- US-B1- 6 200 937

## Description

### BACKGROUND OF THE INVENTION

This application claims the benefit under 35 U.S.C. § 119(e) of earlier filed and copending U.S. Provisional Application No. 60/341,810, filed December 21, 2001 and U.S. Provisional Application No. 60/369,219, filed April 1, 2002, the contents of each of which are incorporated by reference herein.

### 1. Technical Field

The present invention relates to aqueous free flowing compositions. In particular, the invention relates to surfactant compositions which have free-flowing non-Newtonian shear thinning properties.

### 2. Background of Related Art

Many properties are desirable in personal care surfactant compositions such as, for example, shampoos, body cleansers, and compositions for delivering cosmetic agents or topical therapeutic agents. These properties range from improved compositional features to enhanced rheological behavior.

Known surfactant systems generally comprise a primary surfactant, which acts as a cleanser, and a secondary surfactant, which is typically a foam booster or stabilizer. The function of the primary surfactant is to confer cleansing properties to the formulation through the process of detergency. The primary surfactant is, in most cases, anionic in nature to give optimum detergent effects. Alkyl sulfates and alkyl ether sulfates are typical primary surfactants.

The function of the secondary surfactant can be to either boost or stabilize the foam, build viscosity, and/or mitigate irritancy of the primary cleansing surfactant. Secondary surfactants are normally drawn from two classes, nonionic and amphoteric/ zwitterionic surfactants. Commonly used nonionic surfactants include amine oxides and alkyl polysaccharides. The most commonly used amphoteric/zwitterionic surfactants are n-alkyl betaines, n-alkylamido betaines, carboxyamphoglycinates, and hydroxy sultaines.

When anionic compositions are used as shampoos for cleansing hair, it is desirable to treat the hair with a conditioning agent. Hair conditioners can be applied after shampooing to improve the condition and appearance of the hair. The conditioner is normally left on the hair for 1-2 minutes before being rinsed off. Conditioners normally contain cationic surfactants, which are absorbed onto the hair via attraction to the anionic charges on the hair shaft. The most popular class of cationic surfactants in conditioners are the quaternary ammonium compounds.

Today there are a number of products that offer cleansing and conditioning from the same bottle (2 in 1 shampoo and conditioner). The conditioning components used in 2 in 1 shampoos are generally silicones, fatty alcohols, hydrolyzed proteins, cationic polymers and ethoxylated alkyl amines. Cationic quaternary ammonium compounds are not used in these formulations because of incompatibility issues with the primary anionic surfactant used in the shampoo system, the main issue being that the anionic and cationic surfactants form an insoluble precipitate. The conditioning performance achieved with 2 in 1 shampoo/conditioner products is inferior to the application of a separate shampoo and conditioner.

A long-standing compositional challenge has been the desirability of having a composition containing an anionic surfactant for cleansing purposes and a cationic surfactant for conditioning purposes. However, inclusion of both in a single system has been fraught with difficulty. This difficulty has been repeatedly noted in the literature. For example, U.S. Patent No. 4,001,394 notes that cationic surfactants, including quaternary ammonium salts such as halides and sulfates, cannot be successfully included in anionic formulations. As recently as May 2000, a leading shampoo manufacturer documented in European Patent Application EP 1080714A2 that "it is well known that the formulation of such "two-in-one" conditioning (e.g., shampoos with anionic surfactants and cationic conditioning agents) is difficult due to the inherent incompatibility between the cleansing anionic surfactants and the cationic conditioning agents."

Proposed solutions to this problem have included the use of specially created quaternary ammonium derivatives that exhibit improved water solubility and reduced propensity to precipitate out of solution (see U.S. Patent No. 4,069,347 or the use of an anionic cross-linked polymeric suspending agent in order to prevent incompatibility issues (see U.S. Patent No. 5,114,706). However, the solutions to date have typically resulted in products which have reduced cleansing or conditioning properties, or have required the addition of special components.

Other properties that are desirable in personal care surfactant compositions include having a composition that pours easily yet has an appearance of richness by "heaping" in a lotion-like manner upon dispensing, and having a composition with the capacity to suspend water insoluble particles, such as active agents, moisturizing agents and the like. These features relate to the rheological behavior of the surfactant compositions.

The rheological behavior of all surfactant solutions, including personal care compositions, is believed to be dependent on their microstructure, i.e., the shape and concentration of micelles or other self-assembled structures in solutions. Micelles are not necessarily spherical and may, for example, exist as cylindrical or discoidal micelles. At higher concentrations, more ordered liquid crystal phases such as lamellar phases, hexagonal phases or cubic phases may form. Surfactants can take on organized phases above the critical micellar concentration or CMC. (The CMC is defined as the concentration of a surfactant at which it begins to form micelles in solution.) Thus, the rheology of the phase is very important when considering the usefulness of the surfactant system.

The rheology of surfactant systems can be described in terms of Newtonian and non-Newtonian viscosities. The rheology of a Newtonian surfactant is described as having a viscosity that is independent of the shear rate (i.e., the system will have the same viscosity as different levels of shear are applied). The rheology of a non-Newtonian surfactant system is described as having a viscosity that is dependent on the shear rate. For a non-Newtonian shear thinning surfactant system, viscosity will be reduced as the shear rate is increased. This non-Newtonian rheological behavior effectively allows the suspension of undissolved solids, liquids and gases.

General methods for preparing lamellar phase systems can be found in the art. The first commercial application of lamellar based formulations was by Messenger et al. (see U.S. Patent No. 4,659,497) who developed laundry cleaning formulations where high levels of soluble builders, such as STPP and citrate salts, were used to form the lamellar phase and subsequently suspend insoluble or undissolved builders such as zeolite.

U.S. Patent No. 5,556,628 discloses free flowing non-Newtonian shear thinning cosmetic preparations. The compositions disclosed by Derian are based on specified surfactant mixtures including an anionic surfactant, sodium lauryl ether sulfate (also known as sodium laureth sulfate), and identified co-surfactants and electrolytes.

Non-Newtonian shear thinning formulations can be prepared following the teachings of U.S. Patent No. 5,556,628. However, while the subject formulations demonstrate good room temperature (25° C) and elevated temperature (45° C) viscosity stabilities, they may not exhibit optimum performance under all conditions (e.g. freeze/thaw).

The primary focus for these types of cosmetic applications has been body wash products where high surfactant to water ratios are employed to induce the lamellar structure, which in turn is used to suspend high levels of conditioning oils and emollients (see e.g., WO 98/13022 and WO 98/1171).

Other disclosures suggest the use of fatty acid structurants to stabilize lamellar phase systems (see e.g., U.S. Patent Nos. 6,150,312, 5,952,286 and 5,962,395). The inherent disadvantage of such systems requiring fatty acid ingredients is that fatty acids form insoluble salts (Ca⁺² and Mg⁺² salts) in hard water, which leave an undesirable residue on surfaces such as hair, skin, hard surfaces, etc. This residue is particularly unwanted in shampoo formulations where it will cause dulling of the hair and will act as a foam depressant that negatively impacts high foaming cosmetic formulations such as hair shampoos and body washes.

Moreover, it is difficult to maintain the stability and integrity of other known systems, particularly under freeze/thaw conditions.

Hence, it would be desirable to find a personal care free-flowing non-Newtonian shear thinning surfactant composition that has good stability properties at low temperatures. Further, it would be desirable to develop a stable, simple surfactant system comprising a mixture of anionic and cationic surfactants.

The present invention provides an aqueous free flowing composition comprising:
(a) from 7% to 20% by weight of at least one anionic surfactant containing a sulfate of an alcohol derived from a linear alcohol or a branched synthetic alcohol, which alcohol may be alkoxylated;
(b) from 2% to 6% by weight of at least one cationic surfactant having the formula (i) 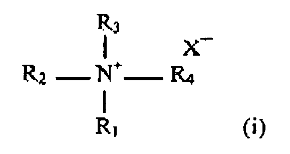 wherein R₁, R₂, R₃ and R₄ are each independently hydrogen or an organic group, with the proviso that at least one of the R groups is not hydrogen, X represents an anion;
(c) an amphoteric surfactant; and
(d) water,
   wherein the total amount of all surfactants in the composition is from 8% to 30%, and further provided that at least one of the anionic surfactant (a) or the cationic surfactant (b) has at least one unsaturated aliphatic group or a branched aliphatic group; and wherein the composition possesses non-Newtonian shear thinning properties and a stable viscosity under at least one freeze/thaw cycle.

The compositions of the invention have improved low temperature stability, can provide enhanced delivery of a cationic surfactant to skin and hair, and have good flash foaming properties as compared to previously known personal care compositions. The compositions form a stable free-flowing composition, which may suspend water insoluble solids or water insoluble liquids such as emollients. Surprisingly, it has been discovered that some mixtures of the anionic and the cationic surfactants in die compositions of the present invention do not form precipitates.

The free-flowing composition may further comprise at least one of a zwitterionic or nonionic surfactant.

The method for making the free-flowing compositions of the invention involves admixing into an aqueous medium, under stirring, an anionic surfactant and a cationic surfactant and an amphoteric surfactant in such amounts as to impart non-Newtonian shear thinning properties thereto. Heat may be applied if necessary. The method may further include incorporation into the composition, e.g., by incorporating into the mixture of the aqueous medium and the cationic and anionic surfactants, at least one water-insoluble particle or partially insoluble component. Other typical ingredients included in this type of formulation include, for example, preservatives, dyes, fragances, chelating agents, etc. Optionally, one or more benefit agents may also be included in the mixture.

The compositions of the invention may be used to suspend agents useful in skin and hair care treatments including, but not limited to, UV absorbers, hair conditioning agents, hair and skin conditioning agents for use in child care formulations, skin conditioning agents, anti-bacterial agents, styling polymers for hair and skin care formulations (including rinse off applications such as shampoos), conditioning polymers for hair and skin care formulations, precipitated conditioning polymers for enhanced active delivery to hair and skin, conditioning polymers possessing high molecular weights and/or cationic charge densities for hair and skin care formulations, surfactants usually associated with solid formulations (such as cocoyl isethionates), and swellable polymers which hydrate only on application. The compositions of the invention may also be used in the preparation of stable, multi-phase personal care formulations, including those with colored stripes found in body washes, hair shampoos, skin cleansers, child care formulations including shampoos and body products, facial washes, and skill treatments.

### BRIEF DESCRIPTION OF THE FIGURES

FIG. 1 is a chart showing the relationship between cationic surfactant concentration and the formation of opaque composition characteristics in a free-flowing composition;
FIG. 2 is a graph showing total work results of wet combing test;
FIG. 3 is a graph showing detangling (peak load) results of wet combing test;
FIG. 4 is a graph showing change in dry comb after treatment;
FIG. 5 is a graph showing detangling of dry hair test results; and
FIG. 6 is a graph showing change in static charge after treatment test results.

The compositions of this invention are free-flowing systems which are capable of suspending dissolved solids or partially soluble materials and enhancing the inclusion of materials with limited solubility in the composition. These compositions are suited for personal care compositions such as shampoos, bath products, lotions, gel soaps, exfoliating gels, creams, and delivery systems for cosmetics or topical therapeutic agents and similar agents. Additionally, these compositions are useful for laundry formulations, lubricity and anti-corrosion metal-working applications, and for cleaners used in sanitizing hard surfaces.

Without wishing to be bound by theory, the inventors believe that in some examples the compositions of the invention may have a lamellar structure. In any event, the compositions of the invention have free-flowing non-Newtonian shear thinning properties and the ability to suspend components such as water-insoluble particles and partially insoluble components (which are known characteristics of lamellar phase surfactant compositions).

As used herein, a "surfactant" is defined as generally referring to surface active agents including all anionic, nonionic, amphoteric/zwitterionic and cationic surface active agents and various mixtures thereof. At least one of the anionic surfactants and the cationic surfactants has at least one unsaturated aliphatic group or a branched aliphatic group. In some embodiments, a salt of trideceth sulfate is the preferred anionic surfactant.

Except in the examples or where otherwise explictly indicated, all numbers in this disclosure indicating amounts or ratios of materials or conditions of reactions, physical properties of materials and/or use, are understood to be modified by the word "about". Where the weight of a surfactant is utilized in this disclosure, weight shall be understood to mean the weight of active surfactant, with the exception of the examples in the tables.

The amount of anionic surfactant, either from a single anionic surfactant or from a mixture of anionic surfactants, is from 7% to 20% by weight of the composition.

The amount of cationic surfactant, either from a single cationic surfactant or from a mixture of cationic surfactants, is from 2% to 6% by weight of the composition.

The compositions of the invention are stable under freeze/thaw conditions. As used herein, "stable" is defined as a % drop of no more than 40%, preferably no more than 35%, in the viscosity measured after at least one (1) freeze/thaw cycle, preferably at least four (4) freeze/thaw cycles. A freeze/thaw cycle is a 24 hour period with 12 hours at -10°C and 12 hours at 25°C for the environment immediately surrounding the test sample.

One of the primary advantages of this invention over the previously known compositions is that the compositions of this invention permit the simple combination of anionic, cationic and amphoteric surfactants and do not require a fatty acid structurant as a stabilizer in forming free flowing non-Newtonian, shear thinning compositions. Further, tho free flowing compositions of the present invention have good freeze/thaw stability without requiring the addition of a separate freeze/thaw stabilizer. Good freeze/thaw stability is defined by a percent drop of no more than 40%, preferably no more than 35%, in viscosity of the composition as measured before and after at least one freeze/thaw cycle, preferably at least four freeze/thaw cycles. As used herein, one freeze/thaw cycle is a 24-hour period with 12 hours at -10°C and 12 hours at 25°C for the environment immediately surrounding the sample.

In some embodiments the free flowing composition may further comprise water-insoluble particles or partially insoluble components which are suspended in the composition.

In other embodiments, the free flowing composition may contain more than one anionic surfactant, more than one cationic surfactant, or more than one of both. Some embodiments may include zwitterionic surfactants or nonionic surfactants and mixtures thereof. Other embodiments may include one or more benefit agent in the composition. A "benefit agent" means any active ingredient that is to be delivered into the skin or hair, onto the skin or hair, or both, at a desired location.

As set forth in Table 1 below, illustrates Reference Compositions formed by combining a single anionic surfactant with a single cationic surfactant. In some embodiments, it may be desirable to use a mixture comprising more than one anionic surfactant, more than one cationic surfactant, or both.

**TABLE 1**

| **Reference formulations including an anionic surfactant and a cationic surfactant** | | | |
|---|---|---|---|
| **Component** | **Chemical Name** | **Example #1** | **Example #2** |
| Rhodapex EST-30 (Rhodia) | Sodium trideceth sulfate, 30% | 52.0 | ---- |
| Rhodapex ESY (Rhodia) | Sodium laureth sulfate, 25% | ---- | 71.4 |
| DI water | | 42.6 | 17.9 |
| Citric Acid, 50% | | 0.2 | ---- |
| Rhodaquat M-242B/90 (Rhodia) | Cetrimonium Bromide, 99% | 5.2 | ---- |
| Ammonyx KP (Stepan) | Olealkonium Chloride, 50% | ---- | 10.7 |

Table 2 below illustrates compositions according to the invention.

**TABLE 2**

| **Exemplary formulations including an anionic surfactant, a cationic surfactant and an amphoteric or a nonionic surfactant** | | | | | | | | |
|---|---|---|---|---|---|---|---|---|
| **Component** | **Chemical Name** | **Example #3** | **Example #4** | **Example #5** | **Example #6** | **Example #7** | **Example #8** | **Example #9** |
| Rhodepex EST-30 (Rhodia) | Sodium trideceth sulfate, 30% | 53.0 | 49.3 | 53.6 | 53.5 | 51.1 | 49.0 | 48.8 |
| Miranol Ultra L-32 (Rhodia) | Sodium lauroamphocetate 32% | 16.4 | 15.2 | 16.6 | 16.5 | 15.8 | 15.2 | 15.1 |
| DI water | | 24.8 | 23.0 | 25.0 | 25.0 | 23.9 | 22.9 | 22.8 |
| Citric Acid, 50% | | 1.9 | 1.8 | 1.9 | 1.9 | 1.8 | 1.8 | 1.8 |
| Rhodaquat M-242B/99 (Rhodia) | Cetrimonium Bromide, 99% | 3.9 | ---- | ---- | ---- | ---- | ---- | ---- |
| Ammonyx CETAC. (Stepan) | Cetrimonium Chloride, 25% | ---- | 10.7 | ---- | ---- | ---- | ---- | ---- |
| Rhodaquat M-214C/99 (Rhodia) | Myristyl Trimonium Bromide, 99% | ---- | ---- | 2.9 | ---- | ---- | ---- | ---- |
| Ammonyx 4002 (Stepan) | Stearalkonium Chloride, 94% | ---- | ---- | ---- | 3.1 | ---- | ---- | ---- |
| Ammonyx KP (Stepan) | Olealkonium Chloride, 50% | ---- | ---- | ---- | ---- | 7.4 | ---- | ---- |
| Loviquat Mono LS (BASF) | Cocatrimonium Methosulfate, 31.9% | ---- | ---- | ---- | ---- | ---- | 11.1 | ---- |
| Loviquat Mono CP (BASF) | Hydroxyethyl Cetyldimonium Phosphate, 30.8% | ---- | ---- | ---- | ---- | ---- | ---- | 11.5 |

A typical anionic surfactant contains sulfates or sulfonates of alcohols derived from linear alcohols (natural or synthetic) or branched synthetic alcohols. The alcohols may be alkoxylated. The alcohol, if alkoxylated, will typically be a 1 to 4 mole (average) ethoxylate, e.g. laureth (3) sulfate or trideceth (3) sulfate, which are the sulfates of a long chain aliphatic alcohol, ethoxylated with about 3 moles of ethylene oxide. Examples of suitable anionic surfactants include salts of sodium trideceth sulfate, alkyl sulfates, alkyl ether sulfates, pareth sulfates, or mixtures thereof. The salt cation may be an alkaline metal such as sodium or potassium. Alternatively, the cation may be ammonium, triethanolamine, or diethanolamine. An anionic surfactant that has a branched aliphatic group is preferred in some embodiments. An anionic surfactant that has an unsaturated aliphatic group may be desirable in other embodiments.

Cationic surfactants are described as carrying a positive charge, usually on a nitrogen atom in the form of an amine salt or a quaternary ammonium compound, and include monoalkyl amine derivatives, dialkyl amine derivatives, or imidazoline derivatives.

The cationic surfactants may be described by the following general formula: 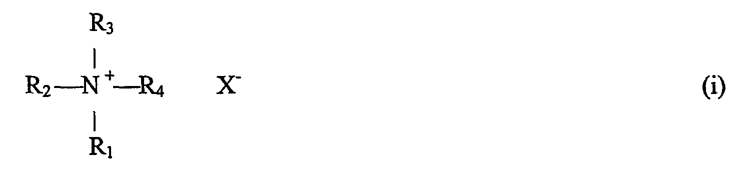 wherein the four R groups, R₁, R₂, R₃ and R₄, are hydrogen, an organic group, or a combination thereof, with the proviso that at least one of the R groups is not hydrogen. X represents a typical anion, which can include chloride, bromide, methosulfate, ethosulfate, lactate, saccharinate, acetate or phosphate. If one to three of the R groups is hydrogen, the compound may be referred to as an amine salt. Some examples of cationic amines include polyethoxylated (2) oleyl/stearyl amine, ethoxylated tallow amine, cocoalkylamine, oleylamine, and tallow alkyl amine. For quaternary ammonium compounds (generally referred to as quats) R₁, R₂, R₃, and R₄ may be the same or different, but may not be hydrogen. In one embodiment, R₁, R₂, R₃, and R₄ are C₈-C₂₄ branched or linear, saturated or unsaturated aliphatic chains, which may comprise additional functionality such as, for example, fatty acids or derivatives thereof, including esters of fatty acids and fatty acids with alkoxylated groups; alkyl amido groups; aromatic rings; heterocyclic rings; phosphate groups; epoxy groups; and hydroxyl groups. The nitrogen atom may also be part of a heterocyclic or aromatic ring system, e.g., cetethyl morpholinium ethosulfate or steapyrium chloride. See International Cosmetic Ingredient Dictionary and Handbook, eighth edition, 2000, Volume 2, p. 1703.

Examples of quaternary ammonium compounds of the monoalkyl amine derivative type include:
Cetyl trimethyl ammonium bromide, also known as CETAB or cetrimonium bromide 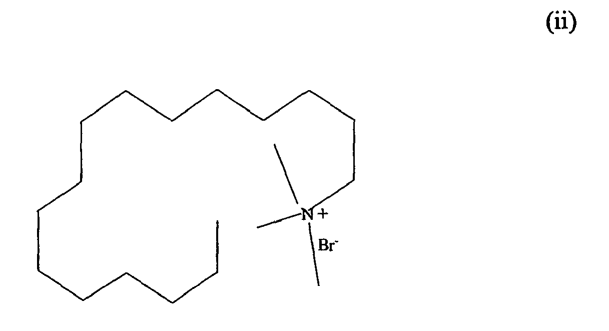
Cetyl trimethyl ammonium chloride, also known as cetrimonium chloride 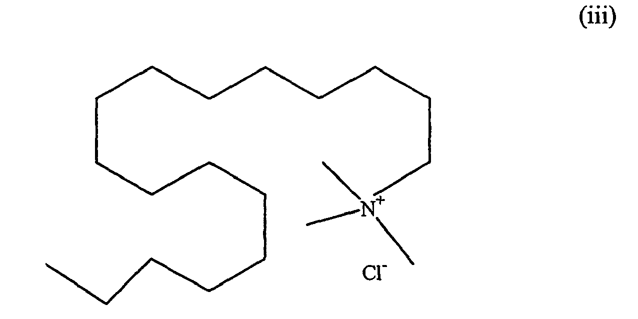
Myristyl trimethyl ammonium bromide, also known as myrtrimonium bromide or Quaternium-13 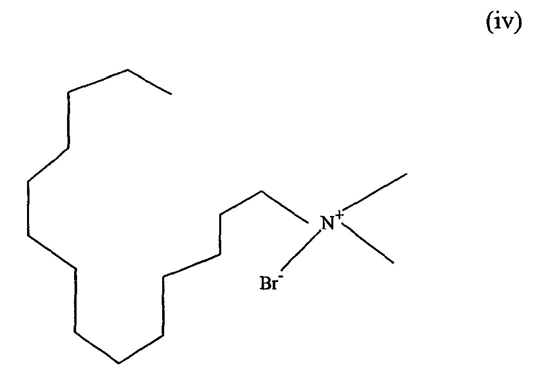
Stearyl Dimethyl Benzyl Ammonium chloride, also known as stearalkonium chloride 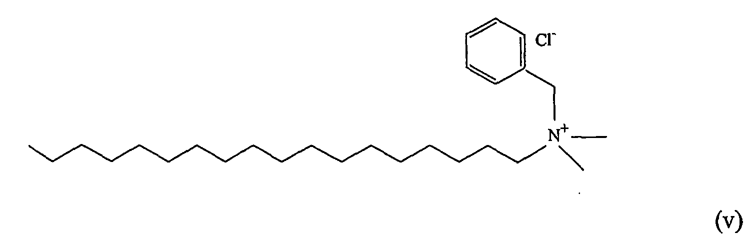
Oleyl Dimethyl Benzyl Ammonium chloride, also known as olealkonium chloride 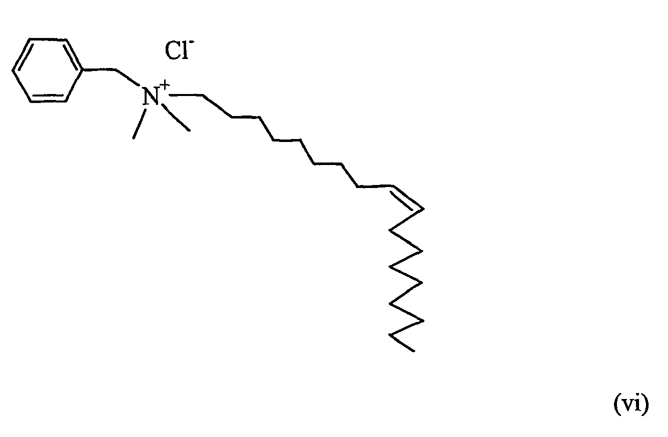
Lauryl/myristryl Trimethyl Ammonium Methosulfate, also known as cocotrimonium methosulfate 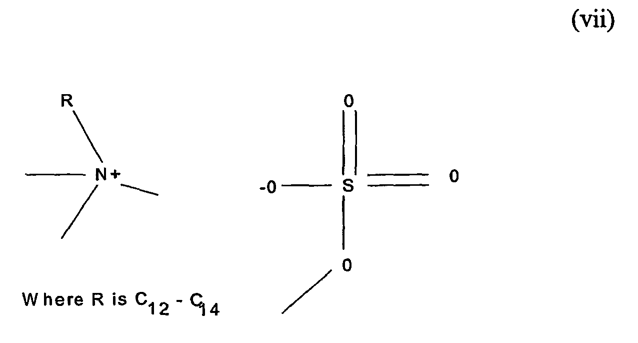
Cetyl-dimethyl-(2)hydroxyethyl ammonium dihydrogen phosphate, also known as hydroxyethyl cetyldimonium phosphate

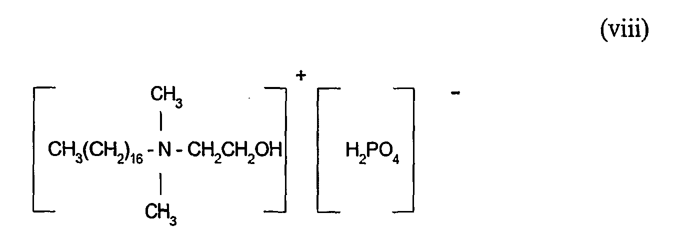

Other cationic surfactants which may be used include, but are not limited to, babassuamidopropylkonium chloride, cocotrimonium chloride, distearyldimonium chloride, wheat germ-amidopropalkonium chloride, stearyl octyldimonium methosulfate, isostearaminopropal-konium chloride, dihydroxypropyl PEG-5 linoleaminium chloride, PEG-2 stearmonium chloride, Quaternium 18, Quaternium 80, Quaternium 82, Quaternium 84, behentrimonium chloride, dicetyl dimonium chloride, behentrimonium methosulfate, tallow trimonium chloride and behenamidopropyl ethyl dimonium ethosulfate.

Alternatively, quaternary ammonium compounds of the group commonly referred to as dialkyl amine derivatives may be used in some embodiments. These compounds include, for example, distearyldimonium chloride, dihydrogenated palmoylethyl hydroxyethylmonium methosulfate, dipalmitoylethyl hydroxyethylmonium methosulfate, dioleoylethyl hydroxyethylmonium methosulfate, and hydroxypropyl bisstearyldimonium chloride.

Quaternary ammonium compounds of the group commonly referred to as imidazoline derivatives may also be used. These compounds include, for example, isostearyl benzylimidonium chloride, cocoyl benzyl hydroxyethyl imidazolinium chloride, cocoyl hydroxyethylimidozolinium PG-chloride phosphate, Quaternium 32, and stearyl hydroxyethylimidonium chloride.

Mixtures of cationic surfactants may also be used in some embodiments. The amount of active cationic surfactant, either from a single cationic or from multiple cationics is from 2% to 6% by weight of the composition. At least one of the cationic surfactants or anionic surfactants has at least one unsaturated aliphatic group or a branched aliphatic group.

The amount of water in the composition vanes, but in general an amount is used such that the total percentage of all other ingredients plus the percentage of water equals 100% by weight.

In any event, the compositions of the invention have free-flowing non-Newtonian shear thinning properties and the ability to suspend components, which are known characteristics of lamellar phase surfactant compositions.

The are flowing composition of the invention may be able to suspend large particles, including particles up to 1 micron and larger, because of the composition's high zero shear viscosity, yet the composition still pours due to its shear thinning properties. The free flowing nature of the composition may be determined visually and the composition can be distinguished by the fact that it has a characteristic shear thinning behavior that can be measured rheologically. The appearance can include translucence, semi-translucence or opaque, and can often include a slightly bluish tint, all of which are detectable by the human eye.

Centrifuging may be used in some exemplary embodiments to determine if the system contains a single phase or multiple phases. Typically a 2 ml sample is centrifuged at 20,000 g (g force) and ambient temperature for 15 minutes.

The free-flowing non-Newtonian shear thinning composition may further comprises an amphoteric surfactant and optionally a zwitterionic surfactant, a nonionic surfactant, or a mixture of such surfactants. Amphoacetates, such as sodium lauroamphoacetate, or diamphoacetates, are preferred in some embodiments. Amphoacetates and diamphoacetates may also be used. Amphoacetates generally conform to the following formula: 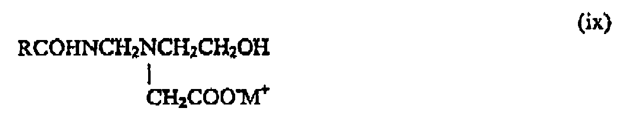 and diamphoacetates generally conform to the following formula: 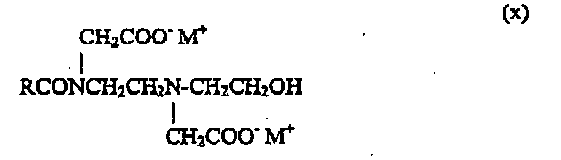 where R is an aliphatic group of 8 to 18 carbon atoms, and M is a cation such as sodium, potassium, ammonium, or substituted ammonium. Sodium lauroamphoacetate, sodium cocoamphoactetate, disodium lauroamphoacetate, and disodium cocoamphodiacetate are preferred in some embodiments.

Other amphoteric/zwitterionic surfactants which may be used in this invention include at least one acid group in their structure. This acid group may be a carboxylic or a sulphonic acid group. The amphoteric/zwitterionic surfactants include a quaternary nitrogen and therefore are quaternary amido acids. They typically further include an alkyl or alkenyl group of 7 to 18 carbon atoms. They will usually comply with the overall structural formula: 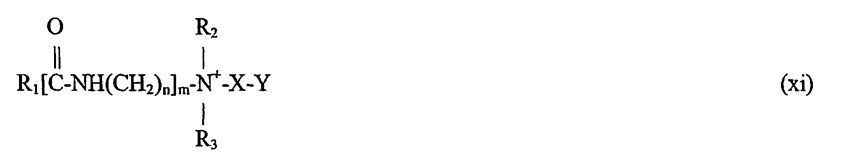 where R₁ is alkyl or alkenyl of 7 to 18 carbon atoms; R₂ and R₃ are each independently alkyl, hydroxyalkyl or carboxyalkyl of 1 to 3 carbon atoms; n is 2 to 4; m is 0 to 1; X is alkylene of 1 to 3 carbon atoms optionally substituted with hydroxyl; and Y is --CO₂ -- or --SO₃--.

Suitable amphoteric/zwitterionic surfactants within the above general formula include simple betaines of the formula: 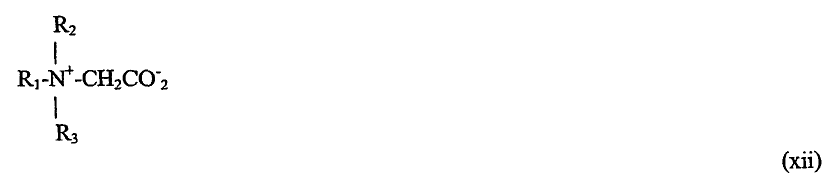 and amido betaines of the formula: 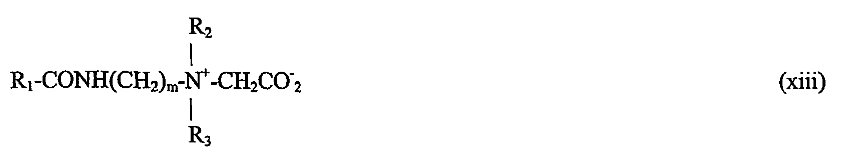 where m is 2 or 3. In both of the above formulae (xii and xiii), R₁, R₂ and R₃ are as defined previously for formula (xi). R₁ may in particular be a mixture of C₁₂ and C₁₄ alkyl groups derived from coconut so that at least half, preferably at least three quarters, of the R₁ groups have 10 to 14 carbon atoms. R₂ and R₃ are preferably methyl.

Further possible amphoteric and/or zwitterionic surfactants are sulphobetaines of the formulae 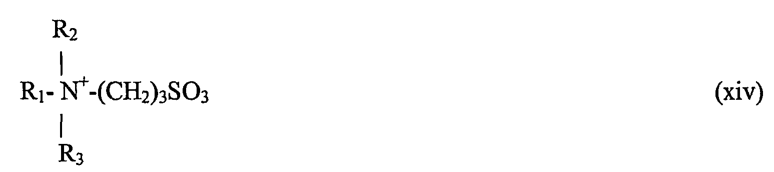 or 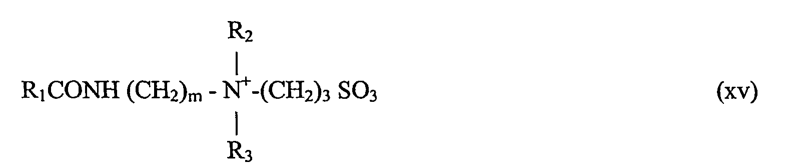 where R₁, R₂ and R₃ are as defined previously for formula (xi), m is 2 or 3, or variants of these in which --(CH₂)₃ SO₃ is replaced by

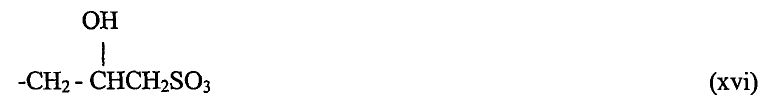

Addition of such amphoteric/zwitterionic surfactants at a pH consistent with the isoelectric point of the amphoteric surfactant may reduce the amount of the cationic surfactant needed in forming a free flowing system.

The surfactant system may also optionally include a nonionic surfactant. Suitable nonionic surfactants include the reaction products of compounds having a hydrophobic group and a reactive hydrogen atom, for example the products obtained by reacting aliphatic alcohols, acids, alkanolamides or alkyl phenols with alkylene oxides, especially ethylene oxide alone or in combination with propylene oxide. Specific nonionic surfactants include the condensation products of alkyl (C₆ -C₂₂) phenols and ethylene oxide, the condensation products of aliphatic (C₈ -C₁₈) primary or secondary linear or branched alcohols with ethylene oxide, and products obtained by the condensation of ethylene oxide with the reaction products of propylene oxide and ethylenediamine. Other nonionic surfactants include long-chain amine oxides, long-chain amido amine oxides, glyceryl long-chain acid esters, sorbitan and ethoxylated sorbitan esters, sucrose esters, long chain tertiary phosphine oxides and dialkyl sulphoxides.

The nonionic surfactants may include sugar amides, such as a polysaccharide amide. Specifically, the surfactant may be one of the lactobionamides described in U.S. Patent No. 5,389,279, or one of the sugar amides described in U.S. Patent No. 5,009,814. Both U.S. Patent Nos. 5,389,279 and 5,009,814 are incorporated by reference herein to the extent they are not inconsistent with this application.

Other surfactants which may be used are those described in U.S. Patent No. 3,723,325, and alkyl polysaccharide nonionic surfactants as disclosed in U.S. Patent No. 4,565,647, both of which are also incorporated by reference herein to the extent they are not inconsistent with this application.

Preferred alkyl polysaccharides are alkylpolyglycosides of the formula

R₂ O(Cₙ H₂ₙ O)ₜ (glycosyl)ₓ (xvii)

wherein R₂ is selected from the group consisting of alkyl, alkylphenyl, hydroxyalkyl, hydroxyalkylphenyl, and mixtures thereof, in which the alkyl groups contain from 10 to 18, preferably from 12 to 14 carbon atoms; n is 0 to 3, preferably 2; t is from 0 to 10, preferably 0; and x is from 1.3 to 10, preferably from 1.3 to 2.7. The glycosyl is preferably derived from glucose. To prepare these compounds, the alcohol or alkylpolyethoxy alcohol is formed first and then reacted with glucose, or a source of glucose, to form the glucoside (attachment at the 1-position). The additional glycosyl units can then be attached between their 1-position and the preceding glycosyl unit's 2-, 3-, 4- and/or 6-position, preferably the 2-position.

When used, the amount of nonionic surfactant is typically from 0.5% to 20%, preferably from 0.75% to 10% and most preferably 1% to 3% by weight of the composition, depending on the type of nonionic surfactant used.

Frequently surfactants are sold as solutions in water or other solvents which dilute them to less than 100% active surfactant, therefore, as used herein, "active surfactant" means the actual amount of surfactant delivered to the free flowing composition from a commercial surfactant preparation. The total amount of all surfactants in the composition, i.e., the sum of anionic surfactants, cationic surfactants, amphoteric/zwitterionic surfactants and nonionic surfactants, is from 8% to 30% active surfactant and preferably 10% to 25% by weight active surfactant.

The compositions of the present invention can also include an electrolyte. The electrolyte can be added separately to the composition or it can be included as part of one of the other raw materials. The electrolyte preferably includes an anion comprising phosphate, chloride, sulfate or citrate and a cation comprising sodium, ammonium, potassium, magnesium or mixtures thereof. Some preferred electrolytes are sodium or ammonium chloride and sodium or ammonium sulfate.

In some embodiments of the present invention, it is desirable to include water-insoluble particles or partially insoluble components in the free flowing composition. The terms "water-insoluble particles" and "partially insoluble components" mean solid or non-solid entities which are not completely solubilized in the aqueous medium of the subject composition and include either insoluble or partially soluble species. The terms "water-insoluble particles" and "partially insoluble components" are also understood to mean and encompass those situations where the solid or non-solid entities are present at concentrations above their solubility limit and therefore portions thereof remain undissolved. Typically, water-insoluble particles or partially insoluble components can he solid particles, liquid ingredients, gases, or mixtures thereof Some preferred examples of gases include air bubbles. Solid particles could include, for example, solid particles of zinc pyrethione, mica, alumina, silicon pigments, moisturizing beads, natural abrasives, synthetic abrasives (exfoliants) such as polyoxyethylene beads, and apricot seeds. The water-insoluble particles typically have an average particle size from 0.5 to 3,000 microns in diameter. The ability to suspend water-insoluble particles or partially insoluble components is a desirable feature of the free flowing compositions of the present invention.

The water insoluble particles which are liquids include vegetable oils, animal fats, mineral oils, petrolatum, silicone oils, polyalkylsiloxanes, polyalkylarylsiloxanes, and mixtures thereof, and are typically present in an amount ranging from about 0.1% to 25% by weight of the composition and preferably 1% to 15% by weight.

More particularly, these liquid materials may include, for example: vegetable oils such as arachis oil, castor oil, cocoa butter, coconut oil, corn oil, cotton seed oil, olive oil, palm kernel oil, rapeseed oil, safflower seed oil, sesame seed oil and soybean oil; esters of butyl myristate, cetyl palmitate, decyloleate, glyceryl laurate, glyceryl ricinoleate, glyceryl stearate, glyceryl isostearate, hexyl laurate, isobutyl palmitate, isocetyl stearate, isopropyl isostearate, isopropyl laurate, isopropyl linoleate, isopropyl myristate, isopropyl palmitate, isopropyl stearate, propylene glycol monolaurate, propylene glycol ricinoleate, propylene glycol stearate, and propylene glycol isostearate; animal fats of acetylated lanolin alcohols, lanolin, lard, mink oil and tallow; and fatty acids and alcohols of behenic acid, palmitic acid, stearic acid, behenyl alcohol, cetyl alcohol, eicosanyl alcohol and isocetyl alcohol.

Water-insoluble particles or partially insoluble components which are gases, include air bubbles suspended in the free flowing solution.

Fragrances are an example of a partially insoluble component that may advantageously be incorporated in the free flowing composition of the invention in higher concentrations than in prior water solutions.

In some embodiments it is desirable to add one or more benefit agents to the free flowing composition. The free flowing composition facilitates delivery of the benefit agent and may, in some embodiments, be considered to be a delivery system for the benefit agent. A "benefit agent" means any active ingredient that is to be delivered into or onto the skin or hair, or any combination thereof, at a desired location.

Examples of suitable benefit agents include, but are not limited to, cationic conditioners; depigmentation agents; UV absorbers; reflectants; thickening agents; detangling/wet combing agents; film forming polymers; humectants; amino acids and their derivatives; antimicrobial agents; anti-acne agents; anti-aging agents; antiseptics; analgesics; local anesthetics; anti-hair loss agents; hair growth inhibitor agents; inflammation inhibitors; deodorants and anti-perspirants; skin emollients and skin moisturizers; hair conditioners; hair softeners; hair moisturizers; vitamins; tanning agents; skin lightening agents; antifungals such as antifungals for foot preparations; depilating agents; shaving preparations; agents for treatment of dandruff, seborrheic dermatitis and psoriasis; counterirritants; hemorrhoidals; insecticides; sunscreens and the like; and mixtures thereof.

Examples of cationic conditioners include cationic cellulose derivatives such as the polymeric quaternary ammonium salts derived from the reaction of hydroxyethyl cellulose with a trimethylammonium substituted epoxide, cationic guar derivatives such as guar hydroxyropyltrimonium chloride, derivatives of acrylamidopropyl trimonium chloride, polymers derived from the monomer diallyldimethylammonium chloride, the copolymer of diallyldimethylammonium chloride with acrylamide, polyquaternium-47, and mixtures thereof These conditioning agents are typically incorporated in amounts from 0.1% to 7% and preferably in amounts from 0.2% to 5% by weight of the composition.

Examples of suitable reflectants include mica, alumina, calcium silicate, glycol dioleate, glycol distearate, silica, sodium magnesium fluorosilicate, and mixtures thereof.

Examples of suitable UV absorbers include benzophenone, bornelone, PABA (Para Amino Benzoic Acid), butyl PABA, cinnamidopropyl trimethyl ammonium chloride, disodium distyrylbiphenyl disulfonate, potassium methoxycinnamate, and mixtures thereof.

Commercially available thickening agents that are capable of imparting the appropriate viscosity to conditioning shampoo compositions are suitable for use in this invention. Examples of suitable thickening agents include: mono or diesters of polyethylene glycol of the formula

HO-(CH₂CH₂)₂H (xviii)

wherein z is an integer from about 3 to about 200; fatty acids containing from about 16 to about 22 carbon atoms; fatty acid esters of alkoxy polyols; alkoxy derivatives of mono and diesters of fatty acids and glycerine; hydroxyalkyl cellulose; alkyl cellulose; hydroxyalkyl alkyl cellulose; and mixtures thereof. More specifically, suitable thickening agents include, for example, behenalkonium chloride, cetyl alcohol, quaternium 46, PG-hydroxyethyl cellulose, cocodimonium chloride, polyquaternium 6, polyquaternium 7, quaternium 18, PEG-18 glycerol oleate/cocoate, a mixture of acrylates/spirit 50 acrylate copolymer, laureth 3 and propylene glycol, a mixture of cocamidopropylbetaine and glyceryl laurate, a mixture of propylene glycol, PEG 55, and propylene glycol oleate, and mixtures thereof. Preferred thickeners include polyethylene glycol esters, and more preferably PEG-150 distearate.

Suitable detangling/wet combing agents include dioleoylamidoethyl hydroxythylmonium methosulfate, di (soyoylethyl) hydroxyethylmonium methosulfate, hydroxyethyl behenamidopropyl dimonium chloride, olealkonium chloride, polyquaternium 47, stearalkonuim chloride, tricetylmonium chloride, guar hydroxypropyltrimonium chloride, hydroxypropyl guar hydroxypropyltrimonium chloride and mixtures thereof.

Suitable film forming polymers include those that, upon drying, produce a substantially continuous coating or film on the hair, skin, or nails. Examples of suitable film forming polymers include acrylamidopropyl trimonium chloride/acrylamide copolymer; corn starch/acrylamide/sodium acrylate copolymer; polyquaternium 10; polyquaternium 47; polyvinylmethyl/maleic anhydride copolymer; styrene/acrylates copolymers; and mixtures thereof.

Commercially available humectants, which are capable of providing moisturizing and conditioning properties to the cleansing composition are suitable for use in the present invention. The humectant is preferably present in an amount of from 0% to 10%, more preferably from 0.5% to 5%, and most preferably from 0.5% to 3%, based on the overall weight of the composition. Examples of suitable humectants include: water soluble liquid polyols including glycerine, propylene glycol, hexylene glycol, butylene glycol, pentylene glycol, dipropylene glycol, and mixtures thereof, polyalkylene glycols of the formula:

HO-(R"O)_{b}-H (xix)

wherein R" is an alkylene group having from 2 to 4 carbon atoms and b is an integer of from 1 to 10 (such as PEG 4); polyethylene glycol ethers of methyl glucose of the formula:

CH₃-C₆H₁₀O₅-(OCH₂CH₂)ₙ-OH (xx)

wherein c is an integer of from 5 to 25; urea; fructose; glucose; honey; lactic acid; maltose; sodium glucuronate; and mixtures thereof. In a more preferred embodiment, the humectant is glycerine.

Suitable amino acids which may be beneficial to hair and skin and in some cases can be included as conditioning agents in the compositions of the present invention include amino acids derived from the hydrolysis of various proteins as well as the salts, esters, and acyl derivatives thereof. Examples of such amino acids include amphoteric/zwitterionic amino acids such as alkylamido alkylamines; stearyl acetyl glutamate; capryloyl silk amino acids; capryloyl collagen amino acids; capryloyl keratin amino acids; capryloyl pea amino acids; cocodimonium hydroxypropyl silk amino acids; corn gluten amino acids; cysteine; hair keratin amino acids; hair amino acids such as aspartic acid, threonine, serine, glutarnic acid, proline, glycine, alanine, half-cystine, valine, methionine, isoleucine, leucine, tyrosine, phenylalamine, cysteic acid, lysine, histidine, arginine, cysteine, tryptophan, citrulline; other silk amino acids and wheat amino acids; and mixtures thereof.

Suitable proteins which may be beneficial to hair and skin and in some cases can be included as conditioning agents include those polymers that have a long chain, i.e. at least 10 carbon atoms, and a high molecular weight, i.e. at least 1000, and are formed by self-condensation of amino acids. Examples of such proteins include collagen, deoxyribonuclease, iodized corn protein, keratin, milk protein, protease, serum protein, silk, sweet almond protein, wheat germ protein, wheat protein, alpha and beta helix of keratin proteins, hair proteins, such as intermediate filament proteins, high-sulfur proteins, ultrahigh-sulfur proteins, intermediate filament-associated proteins, high-tyrosine proteins, high-glycine tyrosine proteins, tricohyalin, and mixtures thereof.

Examples of suitable vitamins which may be beneficial to hair and skin and in some cases can be included as conditioning agents include vitamin B complex, including thiamine, nicotinic acid, biotin, pantothenic acid, choline, riboflavin, vitamin B6, vitamin B12, pyridoxine, inositol, carnitine; vitamins A,C,D,E,K and their derivatives, such as vitamin A palmitate; and pro-vitamins, e.g., panthenol (pro vitamin B5), panthenol triacetate, and mixtures thereof.

Examples of suitable antibacterial agents for hair and skin care applications include bacitracin, erythromycin, triclosan, neomycin, tetracycline, chlortetracycline, phenol, parachlorometa xylenol (PCMX), triclocarban (TCC), chlorhexidine gluconate (CHG), zinc pyrithione, selenium sulfide and mixtures thereof.

Examples of suitable skin emollients and skin moisturizers include vegetable oils such as arachis oil, castor oil, cocoa butter, coconut oil, corn oil, cotton seed oil, olive oil, palm kernel oil, rapeseed oil, safflower seed oil, sesame seed oil and soybean oil; esters such as butyl myristate, cetyl palmitate, decyloleate, glyceryl laurate, glyceryl ricinoleate, glyceryl stearate, glyceryl isostearate, hexyl laurate, isobutyl palmitate, isocetyl stearate, isopropyl isostearate, isopropyl laurate, isopropyl linoleate, isopropyl myristate, isopropyl pahnitate, isopropyl stearate, propylene glycol monolaurate, propylene glycol ricinoleate, propylene glycol stearate, and propylene glycol isostearate; animal fats such as acetylated lanolin alcohols, lanolin, lard, mink oil and tallow; fatty acids and alcohols of behenic acid, palmitic acid, stearic acid, behenyl alcohol, cetyl alcohol, eicosanyl alcohol and isocetyl alcohol.

Additional skin treatment agents and skin conditioning agents may include salicylic acid, alpha hydroxy acids, vitamins, vitamin complexes, abrasives, silicones, silicone derivatives, polymers, natural oils, synthetic oils, mineral oils, petrolatum, methyl gluceth 10, methyl gluceth 20, chitosan, and mixtures thereof.

Examples of suitable hair conditioners include silicones, silicone derivatives, natural oils, synthetic oils, nonionic surfactants, cationic surfactants, waxes, and polymers. Quaternized compounds such as behenamidopropyl PG-dimonium chloride, tricetylammonium chloride, dihydrogenated tallowamidoethyl hydroxyethylmonium methosulfate, and mixtures thereof, as well as lipophilic compounds such as cetyl alcohol, stearyl alcohol, hydrogenated polydecene, and mixtures thereof, may also be used.

An example of a suitable hair conditioner/softener includes silicone compounds, such as those that are either non-volatile or volatile, or mixtures thereof, and those that are water soluble or water insoluble, or mixtures thereof. Examples of suitable silicones include its derivatives such as organo-substituted polysiloxanes, which are either linear or cyclic polymers of silicone/oxygen monomers and which include cetyl dimethicone, cetyl triethylammonium dimethicone copolyol phthalate, cyclomethicone, dimethicone copolyol, dimethicone copolyol lactate, hydrolyzed soy protein/dimethicone copolyol acetate, silicone quaternium 13, stearalkonium dimethicone copolyol phthalate, stearamidopropyl dimethicone, and mixtures thereof.

Examples of suitable hair moisturizers include panthenyl ethyl ether, phytantriol, and mixtures thereof

Examples of sunscreen agents include butyl methoxydibenzoylmethane, octyl methoxycinnamate, oxybenzone, octocrylene, octyl salicylate, phenylbenzimidazole sulfonic acid, ethyl hydroxypropyl aminobenzoate, menthyl anthranilate, aminobenzoic acid, cinoxate, diethanolamine methoxycinnamate, glyceryl aminobenzoate, titanium dioxide, zinc oxide, oxybenzone, octyl dimethyl PABA (padimate O), red petrolatum, and mixtures thereof.

An example of a suitable tanning agent is dihydroxyacetone.

Examples of skin lightening agents include hydroquinone, catechol and its derivatives, ascorbic acid and its derivatives, and mixtures thereof

Examples of suitable insecticides, including insect repellents, anti-scabies and anti-lice treatments, include permethrin; pyrethrin; piperonyl butoxide; imidacloprid; N,N-diethyl toluamide, which refers to the material containing predominantly the meta isomer, i.e., N,N-diethyl-m-toluamide, which is also known as DEET; compounds of the formula: 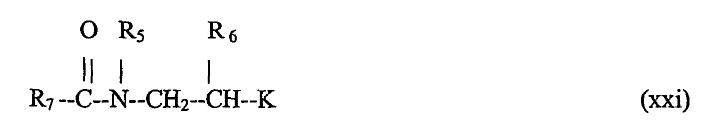 wherein R₅ is a branched or unbranched alkyl group having from about 1 to about 6 carbon atoms, R₆ is H, methyl or ethyl, R₇ is a branched or unbranched alkyl or
alkoxy group having from 1 to 8 carbon atoms, and K is a -CN or a - COOR₈ group, wherein R₈ is a branched or unbranched alkyl group having from

1 to 6 carbon atoms; natural or synthetic pyrethroids, whereby the natural pyrethroids are contained in pyrethrum; the extract of the ground flowers of *Chrysanthemum cinerariae folitan or Chrysanthemum coccineum;* and mixtures thereof. Within the structure of Formula (xxi) are ethyl 3-(N-butylacetamido) propionate, wherein R₇ is a CH₃ group, R₅ is an n-butyl group, R₆ is H, K is COOR₈ and R₈ is ethyl.

An example of an anti fungal for foot preparations is tolnaftate.

Examples of suitable depilating agents include calcium thioglycolate, magnesium thioglycolate, potassium thioglycolate, strontium thioglycolate, and mixtures thereof.

Examples of suitable external analgesics and local anesthetics include benzocaine, dibucaine, benzyl alcohol, camphor, capsaicin, capsicum, capsicum oleoresin, juniper tar, menthol, methyl nicotinate, methyl salicylate, phenol, resorcinol, turpentine oil, and mixtures thereof.

Examples of suitable antiperspirants and deodorants include aluminum chlorohydrates, aluminium zirconium chlorohydrates, and mixtures thereof.

Examples of suitable counterirritants comprise camphor, menthol, methyl salicylate, peppermint oils, clove oils, ichtammol, and mixtures thereof.

An example of a suitable inflammation inhibitor is hydrocortisone.

Examples of suitable hemorrhoidal products include anesthetics such as benzocaine, pramoxine hydrochloride, and mixtures thereof; antiseptics such as benzethonium chloride; astringents such as zinc oxide, bismuth subgallate, balsam Peru, and mixtures thereof; skin protectants such as cod liver oil, vegetable oil, and mixtures thereof.

Examples of suitable benefit agents having therapeutic components that are effective in the treatment of dandruff, seborrheic dermatitis and psoriasis, as well as the symptoms associated therewith, include zinc pyrithione; shale oil and derivatives thereof such as sulfonated shale oil; selenium sulfide; sulfur; salicylic acid; coal tar; povidone-iodine; imidazoles such as ketoconazole, dichlorophenyl imidazolodioxalan, clotrimazole, itraconazole, miconazole, climbazole, tioconazole, sulconazole, butoconazole, fluconazole; miconazolenitrite and any possible stereo isomers and derivatives thereof such as anthralin; piroctone olamine (Octopirox); selenium sulfide; ciclopirox olamine; anti-psoriasis agents such as vitamin D analogs, e.g. calcipotriol, calcitriol, and tacaleitrol; vitamin A analogs such as esters of vitamin A, including vitamin A palmitate, retinoids, retinols, and retinoic acid; corticosteroids such as hydrocortisone, clobetasone, butyrate, clobetasol propionate; and mixtures thereof.

Some preferred benefit agents for treatment of dandruff, seborrheic dermatitis, and psoriasis, as well as the symptoms associated therewith, include sulfonated shale oil, elubiol, 6-(1-piperidinyl)-2-4-pyrimidinediamine-3-oxide, finasteride, ketoconazole, salicylic acid, zinc pyrithione, coal tar, benzoyl peroxide, selenium sulfide, hydrocortisone, sulfur, menthol, praxomine hydrochloride, tricetylammonium chloride, polyquaternium 10, panthenol, panthenol triacetate, vitamin A and derivatives thereof, vitamin B and derivatives thereof, vitamin C and derivatives thereof, vitamin D and derivatives thereof, vitamin E and derivatives thereof, vitamin K and derivatives thereof, keratin, lysine, arginine, hydrolzed wheat proteins, hydrolyzed silk proteins, octyl methoxycinnamate, oxybenzone, minoxidil, titanium dioxide, zinc dioxide, retinol, erythromycin, tretinoin, and mixtures thereof.

Examples of benefit agents suitable for treating hair loss include, but are not limited to, potassium channel openers or peripheral vasodilators such as minoxidil, diazoxide, and compounds such as N"-cyano-N-(tert-pentyl)-N'-3-pyridinyl-guanidine ("P-1075") as disclosed in United States Patent No. 5,244,664, which is incorporated by reference herein; vitamins, such as vitamin E and vitamin C, and derivatives thereof such as vitamin E acetate and vitamin C palmitate; hormones such as erythropoietin; prostaglandins, such as prostaglandin EI and prostaglandin F2-alpha; fatty acids such as oleic acid; diruretics such as spironolactone; heat shock proteins ("HSP"), such as HSP 27 and HSP 72; calcium channel blockers, such as verapamil HCL, nifedipine, and diltiazemamiloride; immunosuppressant drugs, such as cyclosporin and Fk-506; 5 alpha-reductase inhibitors such as finasteride; growth factors such as, EGF, IGF and FGF; transforming growth factor beta; tumor necrosis factor; non-steroidal anti-inflammatory agents such as benoxaprofen; retinoids and derivatives thereof such as tretinoin; cytokines, such as IL-6, IL-1 alpha, and IL-1 beta; cell adhesion molecules such as ICAM; glucocorticoids such as betamethasone; botanical extracts such as aloe, clove, ginseng, rehmannia, swertia, sweet orange, zanthoxylum, *Serenoa repens* (saw palmetto), *Hypoxis rooperi,* stinging nettle, pumpkin seeds, and rye pollen; other botanical extracts including sandalwood, red beet root, chrysanthemum, rosemary, burdock root and other hair growth promoter activators as disclosed in DE 4330597, which is incorporated by reference herein to the extent that it is not inconsistent with this application; homeopathic agents such as Kalium Phosphoricum D2, Azadirachta indica D2, and Joborandi DI; genes for cytokines, growth factors, and male-pattern baldness; antifungals such as ketoconazole and elubiol; antibiotics such as streptomycin; protein inhibitors such as cycloheximide; acetazolamide; benoxaprofen; cortisone; diltiazem; hexachlorobenzene; hydantoin; nifedipine; penicillamine; phenothiazines; pinacidil; psoralens; verapamil; zidovudine; alpha-glucosylated rutin having at least one rutin selected from quercetin, isoquercitrin, hesperidin, naringin, and methylhesperidin; and flavonoids and transglycosidated derivatives thereof which are all disclosed in JP 7002677, which is incorporated by reference herein to the extent that it is not inconsistent with the present application; and mixtures thereof.

Examples of benefit agents suitable for use in inhibiting hair growth include serine proteases such as trypsin; vitamins such as alpha-tocophenol (vitamin E) and derivatives thereof such as tocopherol acetate and tocopherol palmitate; antineoplastic agents, such as doxorubicin, cyclophosphamide, chlormethine, methotrexate, fluorouracil, vincristine, daunorubicin, bleomycin and hydroxycarbamide; anticoagulants, such as heparin, heparinoids, coumaerins, detran and indandiones; antithyroid drugs, such as iodine, thiouracils and carbimazole; lithium and lithium carbonate; interferons, such as interferon alpha, interferon alpha-2a and interferon alpha-2b; retinoids, such as retinol (vitamin A), isotretinoin; glucocorticoids such as betamethasone and dexamethosone; antihyperlipidaemic drugs, such as triparanol and clofibrate; thallium; mercury; albendazole; allopurinol; amiodarone; amphetamines; androgens; bromocriptine; butyrophenones; carbamazepine; cholestyramine; cimetidine; clofibrate; danazol; desipramine; dixyrazine; ethambutol; etionamide; fluoxetine; gentamicin; gold salts; hydantoins; ibuprofen; imipramine; immunoglobulins; indandiones; indomethacin; intraconazole; levadopa; maprotiline; methysergide; metoprolol; metyrapone; nadolol; nicotinic acid; potassium thiocyanate; propranolol; pyridostimine; salicylates; sulfasalazine; terfenadine; thiamphenicol; thiouracils; trimethadione; troparanol; valproic acid; and mixtures thereof.

Examples of suitable anti-aging agents include inorganic sunscreens such as titanium dioxide and zinc oxide; organic sunscreens such as octyl-methyl cinnamates and derivatives thereof; retinoids; vitamins such as vitamin E, vitamin A, vitamin C, vitamin B, and derivatives thereof such as vitamin E acetate, vitamin C palmitate, and the like; antioxidants including beta carotene, alpha hydroxy acid such as glycolic acid, citric acid, lactic acid, malic acid, mandelic acid, ascorbic acid, alpha-hydroxybutyric acid, alpha-hydroxyisobutyric acid, alpha-hydroxyisocaproic acid, atrolactic acid, alpha-hydroxyisovaleric acid, ethyl pyruvate, galacturonic acid, glucoheptonic acid, glucoheptono 1,4-lactone, gluconic acid, gluconolactone, glucuronic acid, glucuronolactone, glycolic acid, isopropyl pyruvate, methyl pyruvate, mucic acid, pyruvic acid, saccharic acid, saccaric acid 1,4-lactone, tartaric acid, and tartronic acid; beta hydroxy acids such as beta-hydroxybutyric acid, beta-phenyl-lactic acid, beta-phenylpyruvic acid; botanical extracts such as green tea, soy, milk thistle, algae, aloe, angelica, bitter orange, coffee, goldthread, grapefruit, hoellen, honeysuckle, Job's tears, lithospermum, mulberry, peony, puerarua, rice, safflower, and mixtures thereof.

Some preferred anti-aging agents comprise retinoids, including retinol and tretinoin, anti-oxidants, alpha-hydroxy acids and beta-hydroxy acids.

Examples of suitable anti-acne agents include, but are not limited to, topical retinoids including tretinoin, isotretinoin, motretinide, adapalene, tazarotene, azelaic acid, retinol; salicylic acid; benzoyl peroxide; resorcinol; antibiotics such as tetracycline and isomers thereof, erythromycin, and the anti-inflammatory agents such as ibuprofen, naproxen, hetprofen; botanical extracts such as alnus, amica, artemisia capillaris, asiasarum root, birth or afterbirth, calendula, chamomile, cnidium, comfrey, fennel, galla rhois, hawthron, houttuynia, hypericum, jujube, kiwi, licorice, magnolia, olive, peppermint, philodendron, salvia, sasa albomarginata; imidazoles such as ketoconazole and elubiol, and mixtures thereof.

Examples of suitable depigmentation agents comprise retinoids such as retinol; kojic acid and its derivatives such as, for example, kojic dipalmitate; hydroquinone and it derivatives such as arbutin; transexamic acid; vitamins such as niacin, vitamin C and its derivatives; azelaic acid; placertia; licorice; extracts such as chamomile and green tea, and mixtures thereof. Retinol, kojic acid, and hydroquinone are preferred.

Other examples of benefit agents comprise allergy inhibitors, anti-wrinkling agents, anti-puritics, antitussives, hair growth promoting agents, antihistamines, anticholinergics, anti-emetics, antiinfectives, vasconstrictors, vasodilators, wound healing promoters, peptides, polypeptides, medicament agents, shaving preparations, poison ivy products, poison oak products, burn products, anti-diaper rash agents, prickly heat agents, herbal extracts, retinal, flavoides, sensates, skin conditioners, hair lighteners, cell turnover enhancers and the like, and mixtures thereof.

The free-flowing Surfactant composition of the present invention may function as a delivery system for a benefit agent. The amount of benefit agent to be combined with the composition of the invention may vary depending upon, for example, the resulting benefit desired and the sensitivity of the user to the benefit agent. Typically the benefit agent is present in a personal care product in an amount, based upon the total weight of the composition or delivery system, from 0.001% to 20%. In a preferred embodiment, the benefit agent is present in an amount from 0.001 % to 0% per total weight of the composition, with a range from 0.001 % to 5% being most preferred. One or more benefit agents may be used in any given embodiment. Selection of which benefit agent to include will depend on the intended end use and the mutual compatibility of the benefit agents selected.

Other components that may be added to the compositions of the invention include those typically added to personal care products or cleaning products, all of which are useful in enhancing the appearance or cosmetic properties of the product. These may include, for example, auxiliary thickeners such as carboxymethylcellulose, magnesium aluminum silicate, hydroxyethylcellulose, methylcellulose, carbopols, glucamides; perfumes; sequestering agents such as tetrasodium ethylenediammetetraacetate (Na₄-EDTA), EHDP, or mixtures thereof, which can be present in varying amounts ranging from 0.01% to 5%, preferably from 0.01% to 3% by weight; and coloring agents, pigments, opacifiers and pearlizers such as zinc stearate, magnesium stearate, TiO₂, EGMS (ethylene glycol monostearate) and Lytron 621 (Styrene/Acrylate copolymer).

Inclusion of antimicrobials may be used advantageously in some embodiments. Such antimicrobials include, for example, 2-hydroxy-4,2'4' trichlorodiphenylether (DP300); preservatives such as dimethyloldimethylhydantoin (Glydant XL1000), parabens, sorbic acid, etc.; antioxidants such as, for example, butylated hydroxytoluene (BHT), and mixtures thereof.

The compositions of the invention may be used to suspend agents useful in skin and hair care treatments including, but not limited to, UV absorbers, hair conditioning agents, hair and skin conditioning agents for use in child care formulations including shampoos and body products, skin conditioning agents, anti-bacterial agents, styling polymers for hair and skin care formulations (including rinse off applications such as shampoos), conditioning polymers for hair and skin care formulations, precipitated conditioning polymers for enhanced active delivery to hair and skin, conditioning polymers possessing high molecular weights and/or cationic charge densities for hair and skin care formulations, surfactants usually associated with solid formulations (such as cocoyl isethionates), and swellable polymers which hydrate only on application.

The composition of the invention may be used to prepare personal care products such as shampoos, skin cleansing preparations, compositions for delivering cosmetic agents or topical therapeutic agents. The compositions of the invention may also be used in the preparation of stable, multi-phase personal care formulations, including those with colored stripes found in body washes, hair shampoos, skin cleansers, kids and baby care formulations, facial washes, and skin treatments. Optionally, the composition of the invention may be used to prepare laundry preparations, preparations for lubricity and anti-corrosion for metal-working applications, and for cleaners used in sanitizing hard surfaces.

In embodiments wherein the composition of the invention is used as a shampoo, the shampoo is applied to wet hair, then the hair is washed in accordance with known practices. More preferably, the composition remains on the hair from

0 to 10 minutes, and preferably from 1 to 5 minutes before rinsing.

The free flowing compositions may be prepared by mixing, with stirring in aqueous medium, at least one anionic surfactant, at least one cationic surfactant, and optionally an amphoteric and/or zwitterionic surfactant, a nonionic surfactant, or a mixture thereof. The surfactants are present in such amounts that the resulting aqueous mixture has non-Newtonian shear thinning behavior and exhibits properties associated with a stable free flowing composition. When an amphoteric surfactant is included, the pH of the composition may be adjusted to maintain the amphoteric' surfactant in the anionic or neutral state. Typically, the pH is adjusted to a range from 5.5 to 6.5.

In some embodiments, solid water-insoluble particles or partially insoluble components may be mixed with the surfactants to disperse the insoluble particles or partially insoluble components. In embodiments where an insoluble oil is used, more vigorous mixing may be desirable to shear the oil into fine droplets in the aqueous mixture. Prewarming the components and mixing at a temperature above ambient temperature may also be desirable. When warming is used, temperature sensitive additives such as perfume and colorants, for example, may be added at the end of the mixing process or after the temperature is returned to near ambient.

In a preferred embodiment, the method for making the free flowing composition of the invention involves admixing into an aqueous medium, under moderate agitation, the surfactants in such amounts as to impart a free flowing non-Newtonian shear thinning phase structure thereto. The surfactants are mixed with moderate agitation until homogeneous, with heating applied as necessary to dissolve the cationic surfactant. In some embodiments, the method further includes incorporation of at least one water-insoluble particle or partially insoluble component, a benefit agent, or mixtures thereof. If these additional components are partially or completely soluble in oil or water, adjustment of the surfactant concentration may be necessary to maintain the free flowing structure.

The following nonlimiting examples are illustrative of the broad range of free-flowing non-Newtonian shear thinning compositions that may be prepared and used in accordance with the present invention.

### EXAMPLE 1

### Cationics as Stabilizers of Anionic Non-Newtonian Systems .

Various cationic surfactants were tested in an anionic/amphoteric base formulation (hereinafter the "base system"). Exemplary formulations and exemplary test results are provided in Tables 3-11 below. The base system shown in Table 3 was first prepared, pH adjusted, and then used in formulating the compositions set forth in Tables 4-10.

**Table 3**

| Base System Formulation | | | |
|---|---|---|---|
| **Component** | **wt (g)** | **w/w%** | **% Act** |
| Sodium Trideceth Sulfate (or TDES) 30%, Rhodapex EST-30 (Rhodia) | 1940 | 55.0 | 16.2 |
| Sodium lauroamphoacetate (or SLAA) 32%; Miranol Ultra L-32 (Rhodia) | 600 | 17.0 | 5.3 |
| Water | 920 | 26.0 | |
| 50% Citric Acid | 69.22 | 2.0 | |
| | 3529.22 | 100.0 | 21.5 |

For the cationic and anionic systems set forth in Tables 4-10 below, a cationic surfactant was added to a measured aliquot of the base system. More specifically, this was accomplished by the following procedure: a measured amount of the base system formulation set forth above in Table 3 was added to a 4-oz bottle, a measured amount of the cationic surfactant solution was then added to the bottle, and the surfactants were mixed by hand for 1 minute by either stirring or shaking the bottle. For samples in which the cationic surfactant was a solid, it was sonicated for 1 hour before mixing to dissolve the solid.

The test solutions were examined for stability and the presence of a free flowing structure by visual observation and centrifuge test results. Upon centrifugation at 20,000 g (g force) and ambient temperature for 15 minutes, a stable free flowing composition had a single phase which was opaque to semi-translucent, and in some instances it also had a slight bluish color. The presence of a clear solution or layer indicated the absence of free flowing structure in that solution or layer.

Exemplary compositions and results are provided below in Tables 4-10. ("Structure" or "structured" means a system that appears shear thinning.).

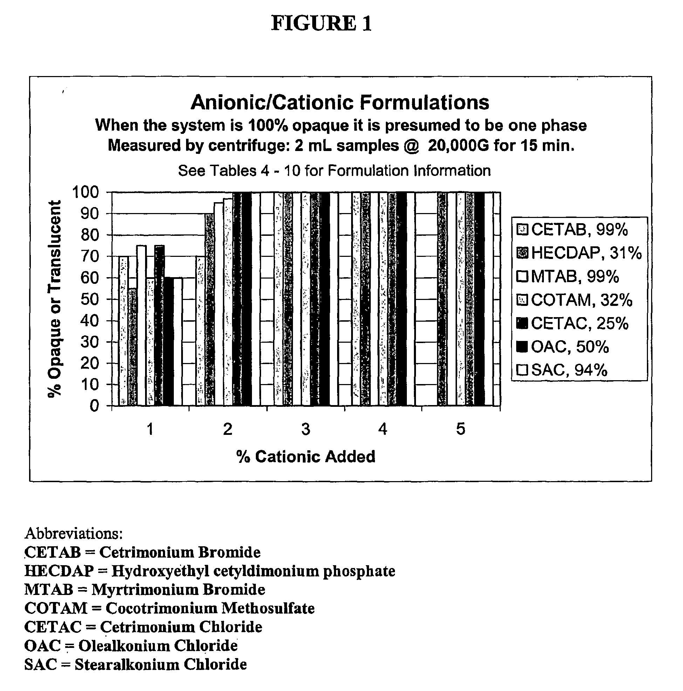

Figure 1 shows the opacity of the formulations of Tables 4-10.

**Table 4**

| Cetrimonium Bromide as Cationic Surfactant | | | | |
|---|---|---|---|---|
| **Sample** | **% Active TDES** | **% Active SLAA** | **% Active cationic** | **Centrifuge Appearance** |
| 1* | 16.10 | 5.31 | 0.50 | 80% clear |
| 2* | 16.02 | 5.28 | 0.99 | 30% clear |
| 3* | 15.94 | 5.26 | 1.48 | 25% clear |
| 4* | 15.86 | 5.23 | 1.96 | 30% clear |
| 5 | 15.78 | 5.21 | 2.44 | 25% clear |
| 6 | 15.70 | 5.18 | 2.91 | stable/structured |
| 7 | 15.63 | 5.16 | 3.38 | stable/structured |
| 8 | 15.55 | 5.13 | 3.85 | stable/structured/bluish |

| | | | | |
|---|---|---|---|---|
| * Comparative | | | | |

**Table 5**

| Cetrimonium Chloride as Cationic Surfactant | | | | |
|---|---|---|---|---|
| **Sample** | **% Active TDES** | **% Active SLAA** | **% Active cationic** | **Centrifuge Appearance** |
| 1* | 15.55 | 5.13 | 0.96 | 25% clear |
| 2* | 14.98 | 4.94 | 1.85 | Stable/opaque |
| 3 | 14.44 | 4.76 | 2.68 | Stable/semi-translucent |
| 4 | 13.94 | 4.60 | 3.45 | Stable/semi-translucent |
| 5 | 13.48 | 4.45 | 4.17 | Stable/semi-translucent |

| | | | | |
|---|---|---|---|---|
| * Comparative | | | | |

**Table 6**

| Myristyl Trimonium Bromide as Cationic Surfactant | | | | |
|---|---|---|---|---|
| **Sample** | **% Active TDES** | **% Active SLAA** | **% Active cationic** | **Centrifuge Appearance** |
| 1* | 16.02 | 5.28 | 0.99 | 25% clear |
| 2* | 15.86 | 5.23 | 1.96 | 5% clear |
| 3 | 15.70 | 5.18 | 2.91 | Stable/remi-translucent |
| 4 | 15.55 | 5.13 | 3.85 | Stable/semi-translucent |
| 5 | 15.41 | 5.08 | 4.76 | Stable/semi-translucent |

| | | | | |
|---|---|---|---|---|
| * Comparative | | | | |

**Table 7**

| Stearalkonium Chloride as Cationic Surfactant | | | | |
|---|---|---|---|---|
| **Sample** | **% Active TDES** | **% Active SLAA** | **% Active cationic** | **Centrifuge Appearance** |
| 1* | 16.01 | 5.28 | 0.99 | 40% clear |
| 2* | 15.84 | 5.23 | 1.96 | Stable/opaque |
| 3 | 15.68 | 5.17 | 2.91 | Stable/semi-translucent |
| 4 | 15.51 | 5.12 | 3.84 | 3.84 Stable/semi-translucent |
| 5 | 15.36 | 5.07 | 4.75 | Stable/semi-translucent |

| | | | | |
|---|---|---|---|---|
| * Comparative | | | | |

**Table 8**

| Olealkonium Chloride as Cationic Surfactant | | | | |
|---|---|---|---|---|
| **Sample** | **% Active TDES** | **% Active SLAA** | **% Active cationic** | **Centrifuge Appearance** |
| 1* | 15.86 | 5.23 | 0.98 | 40% clear |
| 2* | 15.55 | 5.13 | 1.92 | Stable/opaque |
| 3 | 15.26 | 5.03 | 2.83 | Stable/semi-transtucent |
| 4 | 14.98 | 4.94 | 3.70 | Stable/semi-translucent |
| 5 | 14.71 | 4.85 | 4.55 | Stable/semi-translucent |

| | | | | |
|---|---|---|---|---|
| * Comparative | | | | |

**Table 9**

| Cocotimonium Methosulfate as Cationic Surfactant | | | | |
|---|---|---|---|---|
| **Sample** | **% Active TDES** | **% Active SLAA** | **% Active cationic** | **Centrifuge Appearance** |
| 1* | 15.68 | 5.17 | 0.97 | 40% clear |
| 2* | 15.22 | 5.02 | 1.88 | 3% clear |
| 3 | 14.79 | 4.88 | 2.74 | Stable/semi-translucent |
| 4 | 14.37 | 4.74 | 3.55 | Stable/semi-translucent |
| 5 | 13.98 | 4.61 | 4.32 | Stable/semi-translucent |

| | | | | |
|---|---|---|---|---|
| * Comparative | | | | |

**Table 10**

| Hydroxyethyl Cetyldimonium Phosphate | | | | |
|---|---|---|---|---|
| **Sample** | **% Active TDES** | **% Active SLAA** | **% Active cationic** | **Centrifuge Appearance** |
| 1* | 15.68 | 5.17 | 0.97 | 45% clear |
| 2* | 15.22 | 5.02 | 1.88 | 10% clear |
| 3 | 14.79 | 4.88 | 2.74 | Stable/opaque |
| 4 | 14.37 | 4.74 | 3.55 | Stable/semi-translucent |
| 5 | 13.98 | 4.61 | 4.32 | Stable/semi-translucent |

| | | | | |
|---|---|---|---|---|
| * Comparative | | | | |

The effect of using a higher concentration of surfactants (anionic and amphoteric) in the base system, with increasing levels of cationic surfactants, was also tested. As shown below in Table 11, increasing the concentration of the base surfactant system required the use of less cationic surfactant in the system.

**Table 11**

| **SAMPLE** | **% Active TDES** | **% Active SLAA** | **% Active cationic** | **Centrifuge Appearance** |
|---|---|---|---|---|
| 1* | 21.6 | 7.1 | 1.0 | Hazy/does not look structured |
| 2 | 21.4 | 7.1 | 2.0 | Slightly hazy/looks structured |
| 3 | 21.2 | 7.0 | 3.0 | Increased opacity/looks structured |
| 4 | 21.0 | 6.9 | 4.0 | Increased opacity/looks structured |
| 5 | 20.7 | 6.8 | 5.0 | Increased opacity/looks structured |

| | | | | |
|---|---|---|---|---|
| * Comparative | | | | |

Examples of formulations having cationic surfactants and anionic surfactants in the absence of amphoteric surfactants, and the test results for these formulations, are set forth below in Tables 12 and 13.

**Table 12**

| **Sample** | **% Active TDES** | **% Active Cetrimonium Br** | **Centrifuge Appearance** |
|---|---|---|---|
| 1 | 15.68 | 2.91 | 65% clear |
| 2 | 15.60 | 3.38 | 55% clear |
| 3 | 15.53 | 3.85 | 40% clear |
| 4 | 15.45 | 4.31 | 15% clear |
| 5 | 15.38 | 4.76 | stable/opaque |
| 6 | 15.30 | 5.21 | stable/semi-transparent |

**Table 13**

| **Sample** | **% Active TDES** | **% Active Cetrimonium Dr** | **Centrifuge Appearance** |
|---|---|---|---|
| 1* | 19.44 | 1.46 | clear |
| 2 | 18.89 | 2.83 | 5% clear |
| 3 | 18.37 | 4.13 | Stable/opaque |
| 4 | 17.88 | 5.36 | Stable/opaque/bluish |
| 5 | 17.41 | 6.52 | Stable/opaque/bluish |
| 6 | 16.97 | 7.63 | Stable/opaque/bluish |

| | | | |
|---|---|---|---|
| * Comparative | | | |

In general, as demonstrated by the above samples, it was found that using a 75:25 mixture of sodium trideceth sulfate: sodium lauromphoacetate reduced the amount of cetrimonium bromide needed to form the desired free flowing composition system (from 5.5% required for the system lacking the amphoteric surfactant to 3.5% to 4% for the system possessing the amphoteric surfactant).

### EXAMPLE 2

### Hair Conditioning Benefits of Free Flowing Composition of Trideceth Sulfate and Cetrimonium Bromide.

A free flowing composition of trideceth sulfate and cetrimonium bromide was evaluated for wet and dry combing and static reduction performance. The evaluation was performed using a Diastron Tensiometer on wet and dry hair tresses to measure total work of combing and detangling performance.

Hair used in the testing was prepared and evaluated per ADTM 6A. The test samples were composed of control damaged European brown hair of about 2 grams weight and 18 cm in length. Combing measurements were performed using a Diastrom MTT160 Tensiometer. Dry hair was analyzed in a temperature and humidity controlled room of 43-50% relative humidity and 70-72° F. Static measurements were conducted using a Simco Electrostatic Locator type SS-2X; aperture X10; range X2; and distance of tresses to aperture of 3 inches.

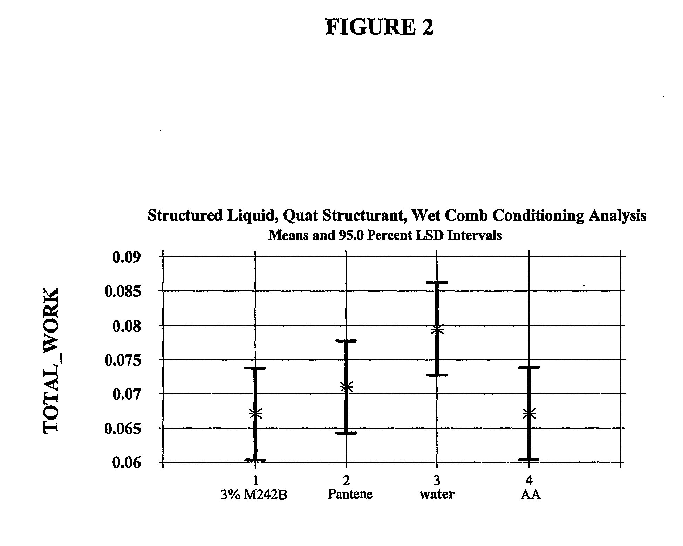

Combing studies were conducted using three formulated systems and water as the untreated control. The formulas were as follows:
1. Sodium Trideceth Sulfate (TDES) with 3% Centrimonium bromide (designated M242B) in Figures 2-6);
2. Commercially available Pantene^{™} 2 in 1 Shampoo manufactured by Proctor & Gamble (designated as Pantene in Figures 2-6);
3. Water; and
4. TDES with sodium lauroamphoacetate (SLAA) (designated as AA in Figures 2-6).

Figure 2 shows total work of wet combing. Total work is the total area under the measured combing curve. All three formulations performed better than water, with M242B and AA performing somewhat better than the commercially available 2 in 1 shampoo.

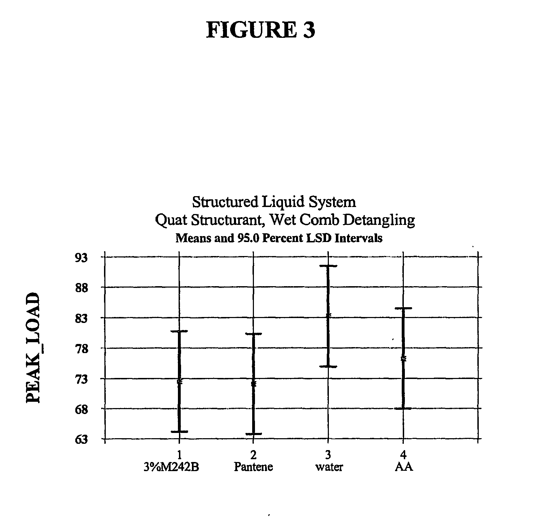

Figure 3 shows results for the detangling and wet combing testing. Detangling is calculated by comparing values of highest peak registered in each individual combing study. Detangling is a measure of the treatment's ability to reduce peak load. All three formulations performed better than water, with M242B and the commercially available 2 in 1 shampoo performing about the same and slightly better than AA.

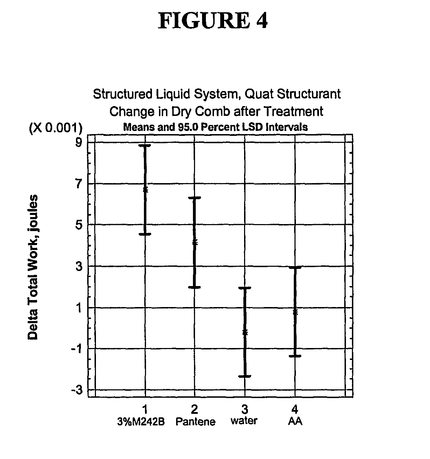

The change (delta) of total work of dry comb results is shown in Figure 4. For dry combing, values are reported as change in combing before and after treatment, hence higher values indicate better performance. As Figure 4 shows, M242B is significantly better than water and AA, and notably better than the commercially available 2 in I shampoo directionally.

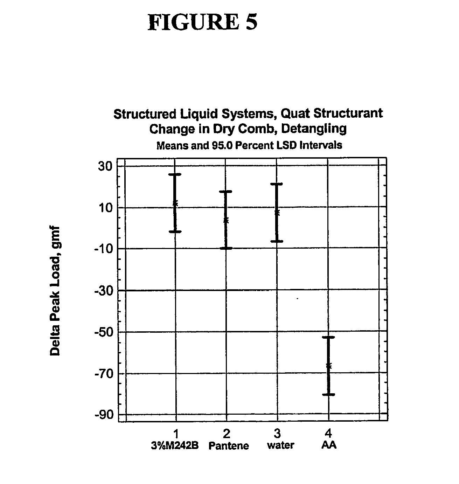

Figure 5 shows results for detangling of dry hair tests. The AA formulation produced results significantly less favorable than the other two formulations and water.

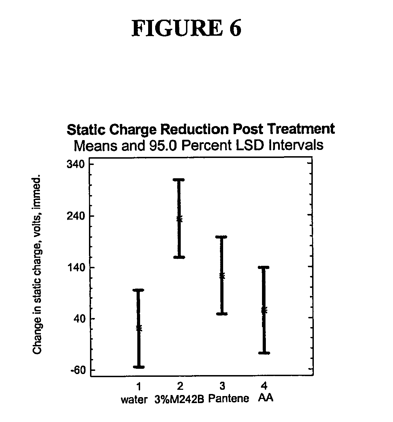

Figure 6 shows results of the static charge test. Measurements were taken both before and after treatment and the difference reported; hence, higher values indicate better performance. M242B was significantly better than water and AA, and directionally better than the commercially available 2 in 1 shampoo.

Those persons skilled in the art will appreciate that the present invention is susceptible to a broad utility and application. Accordingly, while the present invention has been described herein in detail in exemplary embodiments, it is to be understood that this disclosure is only illustrative and exemplary of the present invention and is made merely for purposes of providing a full and enabling disclosure of the invention.

## Claims

1. An aqueous free flowing composition comprising;
(a) from 7% to 20% by weight of at least one anionic surfactant containing a sulfate of an alcohol derived from a linear alcohol or a branched synthetic alcohol, which alcohol may be alkoxylated;
(b) from 2% to 6% by weight of at least one cationic surfactant having the formula (i) 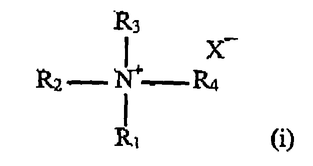 wherein R₁, R₂, R₃ and R₄ are each independently hydrogen or an organic group, with the proviso that at least one of the R groups is not hydrogen, X represents an anion;
(c) an amphoteric surfactant; and
(d) water,
wherein the total amount of all surfactants in the composition is from 8% to 30%, and further provided that at least one of the anionic surfactant (a) or the cationic surfactant (b) has at least one unsaturated aliphatic group or a branched aliphatic group; and wherein the composition possesses non-Newtonian shear thinning properties and a stable viscosity under at least one freeze/thaw cycle.

2. The composition of claim 1, further comprising a surfactant selected from zwitterionic surfactants, nonionic surfactants and combinations thereof.

3. The composition of claim 1, wherein the at least one anionic surfactant comprises an alkyl sulfate or alkyl ether sulfate or a salt thereof.

4. The composition of claim 1 wherein the at least one anionic surfactant is sodium trideceth sulfate.

5. The composition of claim 1 wherein the at least one cationic surfactant is selected from cetrimonium chloride, behentrimonium chloride, cocotrimonium chloride, dicetyl dimonium chloride, behentrimonium methosulfate, stearalkonium chloride, tallow trimonium chloride, cetrimonium bromide, behenamidopropyl ethyl dimonium ethosulfate, distearyldimonium chloride, myristyl trimonium bromide, stearalkonium chloride, olealkonium chloride, coctrimonium methosulfate, stearyl octyldimonium methosulfate, dihydroxypropyl PEG-5 linoleaminium chloride, Quatemium 18, Quaternium 80, Quaternium 84 and hydroxyethyl cetyldimonium phosphate.

6. The composition of claim 1, further comprising an electrolyte.

7. The composition of claim 6 wherein the electrolyte comprises an anion selected from phosphate, chloride, sulfate and citrate.

8. The composition of claim 6 wherein the electrolyte comprises a cation selected from sodium, ammonium, potassium, magnesium and mixtures thereof.

9. The composition of claim 1 further comprising at least one compound selected from water-insoluble particles, partially insoluble components, and benefit agents.

10. The composition of claim 9 wherein the benefit agent is selected from anti-dandruff agents, pigments or opacifying agents, moisturizing beads, natural abrasives, synthetic abrasives such as polyoxyethylene beads, sunscreen agents, vegetable oils, animal fats, mineral oils, petrolatum, silicone oils, polyalkylsiloxanes, polyalkyarylsiloxanes, esters, fatty acids and alcohols, depigmentation agents, reflectants, UV absorbers, thickening agents, detangling/wet combing agents, film forming polymers, humectants, amino acids and their derivatives, antimicrobial agents, anti-acne agents, anti-aging agents, antiseptics, analgesics, local anesthetics, anti-hair loss agents, hair growth inhibitor agents, inflammation inhibitors, proteins, deodorants and anti-perspirants, agents for treatment of dandruff, seborreheic dermatitis and psoriasis, skin emollients, skin moisturizers, hair conditioners, hair softeners, hair moisturizers, vitamins, tanning agents, skin lightening agents, antifungals, depilating agents, counterirritants, hemorrhoidals, insecticides, sunscreens, and mixtures thereof.

11. A stable, multi phase personal care formulation comprising the composition of claim 1.

12. The personal care formulation of claim 11 wherein the personal care formulation is selected from striped body washes, hair shampoos, skin cleansers, child care formulations, facial washes, and skin treatments.

13. A method for making an aqueous free flowing composition comprising admixing:
(a) at least one anionic surfactant containing a sulfate of an alcohol derived from a linear alcohol or a branched synthetic alcohol, which alcohol may be alkoxylated;
(b) at least one cationic surfactant comprising a quaternary ammonium compound having four hydrocarbon substituent groups;
(c) an amphoteric surfactant; and
(d) water,
in sufficient amounts such that the resulting composition comprises 7% to 20% by weight of component (a), 2% to 6% by weight of component (b) and wherein the total amount of all surfactants in the composition is from 8% to 30% by weight and further provided that at least one of (a) or (b) has at least one unsaturated aliphatic group or a branched aliphatic group and wherein the resulting composition possesses non-Newtonium shear thinning properties and a stable viscosity under at least one freeze/thaw cycle.

## Patentansprüche

1. Wässrige frei fließende Zusammensetzung umfassend:
(a) von 7 bis 20 Gew.-% mindestens eines anionischen Tensids, enthaltend ein Sulfat eines Alkohols, abgeleitet von einem linearen Alkohol oder einem verzweigtkettigen synthetischen Alkohol, wobei der Alkohol alkoxyliert sein kann;
(b) von 2 bis 6 Gew.-% mindestens eines kationischen Tensids mit der Formel (i) 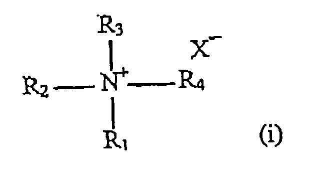 worin R₁, R₂, R₃ und R₄ jeweils unabhängig voneinander Wasserstoff oder eine organische Gruppe sind, mit der Maßgabe, dass mindestens eine der R-Gruppen nicht Wasserstoff ist, X ein Anion ist;
(c) ein amphoteres Tensid, und
(d) Wasser,
wobei die Gesamtmenge aller Tenside in der Zusammensetzung von 8% bis 30% beträgt, und unter der weiteren Maßgabe, dass mindestens eines der anionischen Tenside (a) oder der kationischen Tenside (b) mindestens eine ungesättigte aliphatische Gruppe oder eine verzweigtkettige aliphatische Gruppe aufweist; und wobei die Zusammensetzung nicht-Newton'sches strukturviskoses Verhalten und eine stabile Viskosität unter zumindest einem Gefrier/Tau-Zyklus besitzt.

2. Zusammensetzung nach Anspruch 1, weiterhin umfassend ein Tensid, ausgewählt aus zwitterionischen Tensiden, nichtionischen Tensiden und Kombinationen davon.

3. Zusammensetzung nach Anspruch 1, wobei das mindestens eine anionische Tensid ein Alkylsulfat oder Alkylethersulfat oder ein Salz davon umfasst.

4. Zusammensetzung nach Anspruch 1, wobei das mindestens eine anionische Tensid Natriumtridecethsulfat ist.

5. Zusammensetzung nach Anspruch 1, wobei das mindestens eine kationische Tensid ausgewählt ist aus Cetrimoniumchlorid, Behentrimoniumchlorid, Cocotrimoniumchlorid, Dicetyldimoniumchlorid, Behentrimoniummethosulfat, Stearalkoniumchlorid, Talgtrimoniumchlorid, Cetrimoniumbromid, Behenamidopropylethyldimoniumethosulfat, Distearyldimoniumchlorid, Myristyltrimoniumbromid, Stearalkoniumchlorid, Olealkoniumchlorid, Coctrimoniummethosulfat, Stearyloctyldimoniummethosulfat, Dihydroxypropyl-PEG-5-linoleaminiumchlorid, Quaternium 18, Quaternium 80, Quaternium 84 und Hydroxyethylcetyldimoniumphosphat.

6. Zusammensetzung nach Anspruch 1, des Weiteren umfassend einen Elektrolyt.

7. Zusammensetzung nach Anspruch 6, wobei der Elektrolyt ein Anion umfasst, ausgewählt aus Phosphat, Chlorid, Sulfat und Citrat.

8. Zusammensetzung nach Anspruch 6, wobei der Elektrolyt ein Kation umfasst, ausgewählt aus Natrium, Ammonium, Kalium, Magnesium und Gemischen davon.

9. Zusammensetzung nach Anspruch 1, des Weiteren umfassend mindestens eine Verbindung, ausgewählt aus wasserunlöslichen Teilchen, teilweise unlöslichen Komponenten und Mitteln mit vorteilhaften Eigenschaften.

10. Zusammensetzung nach Anspruch 9, wobei das Mittel mit vorteilhaften Eigenschaften ausgewählt ist aus Antischuppenmitteln, Pigmenten oder Trübungsmitteln, feuchthaltenden bzw. feuchtigkeitsspendenden Perlen, natürlichen Schleifmitteln, synthetischen Schleifmitteln, wie Polyoxyethylenperlen, Lichtschutzmitteln, Pflanzenölen, tierischen Fetten, Mineralölen, Petrolatum, Siliconölen, Polyalkylsiloxanen, Polyalkylarylsiloxanen, Estern, Fettsäuren und Alkoholen, Depigmentierungsmitteln, Reflexionsmitteln, UV-Absorbern, Verdickungsmitteln, Detangling/feuchtigkeitskombinierenden Mitteln, filmbildenden Polymeren, Benetzungsmitteln, Aminosäuren und deren Derivaten, antimikrobiellen Mitteln, Antiaknemitteln, Antialterungsmitteln, Antiseptika, Analgetika, Lokalanästhetika, Mitteln gegen Haarausfall, Haarwachstumshemmmitteln, Entzündungshemmern, Proteinen, Deodorantien und Antitranspirationsmitteln, Mitteln zur Behandlung von Schuppen, seborrhoischer Dermatitis und Psoriasis, hauterweichenden Mitteln, hautbefeuchtenden Mitteln, Haar-Conditioner, haarweichmachenden Mitteln, Haarbefeuchtungsmitteln, Vitaminen, Bräunungsmitteln, hautaufhellenden Mitteln, Antimykotika, Enthaarungsmitteln, Mitteln gegen Reizung, Mitteln gegen Hämorrhoiden, Insektiziden, Lichtschutzmitteln und Gemischen davon.

11. Stabile, Multiphasen-Körperpflegeformulierung, umfassend die Zusammensetzung nach Anspruch 1.

12. Körperpflegeformulierung nach Anspruch 11, wobei die Körperpflegeformulierung ausgewählt ist aus "striped" Körperwaschmitteln, Haarshampoos, Hautreinigern, Kinderpflegeformulierungen, Gesichtsreinigungsmitteln und Hautbehandlungsmitteln.

13. Verfahren zur Herstellung einer wässrigen frei fließenden Zusammensetzung, umfassend das Vermischen von:
(a) mindestens einem anionischen Tensid, enthaltend ein Sulfat eines Alkohols, abgeleitet von einem linearen Alkohol oder einem verzweigtkettigen synthetischen Alkohol, wobei der Alkohol alkoxyliert sein kann;
(b) mindestens einem kationischen Tensid, umfassend eine quaternäre Ammoniumverbindung mit vier Kohlenwasserstoffsubstituentengruppen;
(c) einem amphoteren Tensid; und
(d) Wasser,
in ausreichenden Mengen, so dass die resultierende Zusammensetzung 7 bis 20 Gew.-% an Komponente (a), 2 bis 6 Gew.-% an Komponente (b) umfasst, und wobei die Gesamtmenge an allen Tensiden in der Zusammensetzung von 8 bis 30 Gew.-% beträgt und außerdem unter der Maßgabe, dass mindestens eines von (a) oder (b) mindestens eine ungesättigte aliphatische Gruppe oder eine verzweigte aliphatische Gruppe aufweist, und wobei die resultierende Zusammensetzung nicht-Newton'sches strukturviskoses Verhalten und eine stabile Viskosität unter mindestens einem Gefrier/Tau-Zyklus besitzt.

## Revendications

1. Composition aqueuse fluide comprenant :
(a) de 7 % à 20 % en poids d'au moins un tensioactif anionique contenant un sulfate d'un alcool dérivé d'un alcool linéaire ou d'un alcool synthétique ramifié, l'alcool pouvant être alcoxylé ;
(b) de 2 % à 6 % en poids d'au moins un tensioactif cationique répondant à la formule (i) 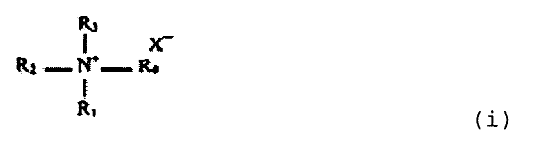 dans laquelle R₁, R₂, R₃ et R₄ représentent chacun indépendamment de l'hydrogène ou un groupe organique, à condition qu'au moins l'un des groupes R ne représente pas un atome d'hydrogène, X représente un anion :
(c) un tensioactif amphotère ; et
(d) de l'eau,
dans laquelle la quantité totale de tous les tensioactifs dans la composition est de 8 % à 30 %, et à condition en outre qu'au moins l'un du tensioactif anionique (a) ou du tensioactif cationique (b) ait au moins un groupe aliphatique insaturé ou un groupe aliphatique ramifié ; et dans laquelle la composition possède des propriétés de fluidification par cisaillement non newtonienne et une viscosité stable pendant au moins un cycle de gel/dégel.

2. Composition selon la revendication 1, comprenant en outre un tensioactif choisi parmi les tensioactifs zwitterioniques, les tensioactifs non ioniques et les combinaisons de ces composés.

3. Composition selon la revendication 1, dans laquelle le au moins un tensioactif anionique comprend un sulfate d'alkyle ou un sulfate d'éther alkylique ou un de leurs sels.

4. Composition selon la revendication 1 dans laquelle le au moins un tensioactif anionique est du sulfate de trideceth de sodium.

5. Composition selon la revendication 1 dans laquelle le au moins un tensioactif cationique est choisi parmi le chlorure de cétrimonium, le chlorure de behentrimonium, le chlorure de cocotrimonium, le chlorure de dicétyldimonium, le méthosulfate de behentrimonium, le chlorure de stéaralconium, le chlorure de suif trimonium, le bromure de cétrimonium, l'éthosulfate de béhénamidopropyléthyldimonium, le chlorure de distéaryldimonium, le bromure de myristyltrimonium, le chlorure de stéaralconium, le chlorure d'oléalconium, le méthosulfate de cocotrimonium, le méthosulfate de stéaryloctyl-dimonium, le chlorure de dihydroxypropyl PEG-5 linoléaminium, le Quaternium 18, le Quaternium 80, le Quaternium 84 et le phosphate d'hydroxyéthylcétyldimonium.

6. Composition selon la revendication 1, comprenant en outre un électrolyte.

7. Composition selon la revendication 6, dans laquelle l'électrolyte comprend un anion choisi parmi un phosphate, un chlorure, un sulfate et un citrate.

8. Composition selon la revendication 6, dans laquelle l'électrolyte comprend un cation choisi parmi le sodium, l'ammonium, le potassium, le magnésium et leurs mélanges.

9. Composition selon la revendication 1 comprenant en outre au moins un composé choisi parmi les particules insolubles dans l'eau, les composants partiellement insolubles, et les agents bénéfiques.

10. Composition selon la revendication 9, dans laquelle l'agent bénéfique est choisi parmi les agents antipelliculaires, les pigments ou les agents opacifiants, les perles hydratantes, les abrasifs naturels, les abrasifs synthétiques tels que les perles de polyoxyéthylène, les agents d'écran solaire, les huiles végétales, les graisses animales, les huiles minérales, le pétrolatum, les huiles de silicone, les polyalkylsiloxanes, les polyalkyarylsiloxanes, les esters, les acides gras et les alcools, les agents de dépigmentation, les agents réflecteurs, les absorbeurs UV, les agents épaississants, les agents de démêlage/peignage humide, les polymères filmogènes, les humectants, les acides aminés et leurs dérivés, les agents antimicrobiens, les agents anti-acné, les agents anti-vieillissement, les antiseptiques, les analgésiques, les anesthésiques locaux, les agents prévenant la chute des cheveux, les agents inhibiteurs de la croissance capillaire, les inhibiteurs de l'inflammation, les protéines, les déodorants et les anti-perspirants, les agents pour le traitement des pellicules, de la parakératose séborrhéïque et du psoriasis, les émollients pour la peau, les hydratants pour la peau, les conditionneurs pour les cheveux, les produits adoucissants pour les cheveux, les hydratants pour les cheveux, les vitamines, les agents tannants, les agents éclaircissant la peau, les antifongiques, les agents dépilatoires, les révulsifs, les hémorroïdaux, les insecticides, les écrans solaires, et leurs mélanges.

11. Formulation stable et à plusieurs phase pour la toilette comprenant la composition selon la revendication 1.

12. Formulation pour la toilette selon la revendication 11, dans laquelle la formulation pour la toilette est choisie parmi les savons liquides pour le corps présentant des bandes, les shampooings capillaires, les nettoyants pour la peau, les formulations de soins pour les enfants, les produits de lavage pour le visage, et les traitements pour la peau.

13. Procédé de préparation d'une composition aqueuse fluide comprenant
(a) au moins un tensioactif anionique contenant un sulfate d'un alcool dérivé d'un alcool linéaire ou d'un alcool synthétique ramifié, l'alcool pouvant être alcoxylé ;
(b) au moins un tensioactif cationique comprenant un composé à base d'ammonium quaternaire ayant quatre groupes de substitution hydrocarbonés ;
(c) un tensioactif amphotère ; et
(d) de l'eau,
en quantités suffisantes pour que la composition résultante comprenne de 7 % à 20 % en poids de composant (a), de 2 % à 6 % en poids de composant (b) et dans laquelle la quantité totale de tous les tensioactifs dans la composition est de 8 % à 30 % en poids et à condition en outre qu'au moins l'un du tensioactif anionique (a) ou du tensioactif cationique (b) ait au moins un groupe aliphatique insaturé ou un groupe aliphatique ramifié et dans laquelle la composition résultante possède des propriétés de fluidification par cisaillement non newtonienne et une viscosité stable pendant au moins un cycle de gel/dégel.
